# EUROPÄISCHE PATENTSCHRIFT

(11) **EP 4 536 326 B1**
(45) Veröffentlichungstag und Bekanntmachung des Hinweises auf die Patenterteilung: **27.05.2026**
(21) Anmeldenummer: 23750913.8
(22) Anmeldetag: 09.06.2023
(51) Int. Cl.: A61M 5/20, A61M 5/28

(54) **SELBSTINJEKTOR**
SELF INJECTOR
AUTO-INJECTEUR

(30) Priorität: 09.06.2022 CH 7042022
(43) Veröffentlichungstag der Anmeldung: 16.04.2025
(73) Patentinhaber: Biorem AG, 6340 Baar (CH)
(72) Erfinder: FELBER, Josef, 8702 Zollikon (CH)
(74) Vertreter: Felber, Josef
(86) Internationale Anmeldenummer: PCT/EP2023/065437
(87) Internationale Veröffentlichungsnummer: WO 2023/237711

(56) Entgegenhaltungen:
- WO-A1-2016/091869
- WO-A2-2009/098502

## Beschreibung

Diese Erfindung betrifft einen Selbstinjektor zum Verabreichen von Medikamenten durch Spritzen durch den Benützer selbst.

Fast die Hälfte aller Medikamente werden heute durch Spritzen verabreicht. Das entspricht in der Grössenordnung jährlich etwa 50 Milliarden Spritzen-Applikationen weltweit. Ein Drittel der Verabreichungen erfolgt oral, der Rest rektal oder über die Augen, Nasen und Ohren. Insulin macht den grössten Anteil der Selbstinjektionen aus. Ein dafür eingesetzter Autoinjektor enthält eine gespeicherte Energie zum Einspritzen des flüssigen Medikaments. Das Wachstum des Marktes für solche vorgefüllte Spritzen wird hauptsächlich durch die steigende Nachfrage nach vorgefüllten Spritzen angetrieben, die auf die wachsende Prävalenz chronischer Krankheiten, technologische Fortschritte, die zunehmende Verbreitung von selbstinjizierenden parenteralen Geräten und unterstützende staatliche Vorschriften (insbesondere Nadelstich-Gesetze) zurückzuführen ist. Darüber hinaus wird das Wachstum des Marktes für vorgefüllte Spritzen durch das wachsende Bewusstsein für die Vorteile vorgefüllter Spritzen bei Patienten und medizinischem Fachpersonal sowie durch die steigende Anzahl von Biologika und Biosimilars auf dem Pharmamarkt unterstützt.

Aus der WO 2011043714 A1 ist zum Beispiel eine Vorrichtung zur Verabreichung von Medikamenten bekannt. Diese umfasst ein längliches röhrenförmiges Gehäuse mit gegenüberliegenden proximalen 10 und distalen 12 Teilen. Weiter ist eine Nadelschutzhülse 20 vorhanden, die verschiebbar und koaxial innerhalb des Gehäuses angeordnet ist. Ein Spritzenträgermechanismus umfasst einen Spritzenträger 36, der verschiebbar innerhalb der Nadelschutzhülse angeordnet ist. Die Spritze 16 umfasst einen Stopfen 92, ein Medikament und eine Nadel und sie ist innerhalb des Spritzenträgers angeordnet ist. Ein erstes Aktivatorelement 56 ist verschiebbar innerhalb des Gehäuses angeordnet und mit der Nadelschutzhülse verbunden. Ein zweites Aktivatorelement 66 ist verschiebbar innerhalb des ersten Aktivatorelements angeordnet. Weiter gibt es einen Antriebsmechanismus, der gleitend innerhalb des zweiten Antriebsmechanismus angeordnet ist, der seinerseits innerhalb des zweiten Aktivatorelements gleitend angeordnet und lösbar mit dem Halteelement und dem zweiten Aktivatorelement verbunden ist. Dieser Antriebsmechanismus kann eine Antriebskraft entwickeln, um den Spritzen-Trägermechanismus in einem ersten Schritt zum Einstechen der Nadel in eine Injektionsstelle und zum Bewegen des Stopfens in einem zweiten Schritt zum Ausstoßen des Medikaments durch die Nadel zu bewegen. Dazu wird der Antriebsmechanismus durch das erste und das zweite Aktivierungselement gesteuert. Diese Vorrichtung weist als Besonderheit ein zweites Aktivatorelement 66 mit Informationsmitteln auf, sodass, nachdem das Medikament vollständig ausgestoßen ist, eine verbleibende Antriebskraft das zweite Aktivatorelement zwingt, in Richtung des distalen Teils des langgestreckten rohrförmigen Gehäuses verschoben zu werden, um eine akustische, visuelle und/oder taktile Rückmeldung an einen Benutzer über eine abgeschlossene Injektion abzugeben.

Weiter ist aus der US 1 0420 898 eine Medikamenten-Abgabevorrichtung mit einer Antriebseinrichtung bekannt, die so konfiguriert ist, dass sie auf einen Medikamentenbehälter einwirkt, um ein Medikament auszustoßen. Dazu weist sie ein Haltemittel auf, das so konfiguriert ist, dass es das Antriebsmittel in einem vorgespannten Zustand hält. Weiter gibt es ein Aktivierungsmittel, das zum Zusammenwirken mit dem Haltemittel zusammenwirkt, um das Antriebsmittel aus dem vorgespannten Zustand freizugeben, wobei die Vorrichtung ferner Rückkopplungsmittel umfasst, die so konfiguriert sind, dass sie sowohl mit dem Haltemittel als auch mit Antriebsmitteln zusammenwirken, um ein hörbares und/oder fühlbares und/oder ein visuelles Signal zu erzeugen, das anzeigt, dass das Medikament vollständig ausgestoßen wurde. US-A-20040054326 beschreibt, besondere anhand der Fig. 1-5 ersichtlich, einen Autoinjektor, umfassend einen Körperteil 21 zur Aufnahme einer Spritze oder Patrone 1 mit einem darin verschiebbar angebrachten Spund mit einem darin gleitend angebrachten Spund zum Ausstoßen einer Dosis; einen Antriebsmechanismus, der Energiespeichermittel 10 in Form einer Spiralfeder enthält und so ausgelegt ist, dass er bei Betätigung eines Auslösers 14 aus einer gespannten Position heraus das Ausstossen einer Dosis bewirkt, wobei der Antriebsmechanismus ein drehbares Kurbeltriebwerk 8 umfasst, das mit einem verschiebbaren Antriebskolben 5 verbunden ist, und die Spiralfeder auf eine Drehkurbelelement 8 wirkt. Die aufbringbare Kraft und der Kraftverlauf ist mit einer Spiralfeder angetrieben aber zu schwach und die vollständige Entleerung der eingelegten Spritze ist kaum gewährbar. WO 2009/098502 A2 zeigt einen Selbstinjektor mit gespeichter Energie für die Applikation eines enthaltenen flüssigen Wirkstoffes mit einem Gehäuse, einer Halterung für das Festhalten einer einzulegenden Spritze mit Zylinder, Dichtzapfen und Nadel, zum hydraulischen Ausstossen des Wirkstoffes durch die Nadel. Es ist eine Torsionsfeder verbaut, die ein Antriebsrad dreht, sodass ein Schwenkhebel und Kolbenschieber bewegt wird, um die Spritze zu verschieben sowie eine Medikamentenababe zu verursachen Der Kolbenschieber wird aber nicht im Injektorgehäuse geführt. WO 2016/091869 A1 zeigt eine Lösung mit einem spannbaren Antriebsrad.

Die Aufgabe der vorliegenden Erfindung ist es angesichts dieses Standes der Technik, einen verbesserten Selbstinjektor zu schaffen, der kostengünstiger herstellbar ist und eine hohe Funktionssicherheit bietet, sodass das sichere Verabreichen eines flüssigen Medikamentes geboten wird, und der aus weniger Teilen als herkömmliche Lösungen besteht, mit einem minimalen Anteil an Teilen aus Metall, und der für den Anwender einfach und narrensicher zu handhaben ist. Insbesondere soll er auch Verletzungen oder Infektionen mit der gebrauchten Spritzennadel sicher verhindern und die Nadel der Spritze nach erfolgter Aktivierung komplett in das Gehäuse zurückziehen, damit sie niemanden verletzen oder infizieren kann.

Die Lösung bietet ein Selbstinjektor mit gespeicherter Energie für die Applikation eines enthaltenen flüssigen Wirkstoffes mit einem Gehäuse, einer Halterung für das Festhalten einer einzulegenden Spritze mit Zylinder, Dichtzapfen und Nadel, zum hydraulischen Ausstossen des Wirkstoffes durch die Nadel, der sich dadurch auszeichnet, dass der Kolben von einem längs einer Schiene im Gehäuse geführten Kolben-Schieber durch einen an demselben angelenkten Schwenkhebel betätigbar ist, und das andere Ende des Schwenkhebels an einem schwenkbaren Federschenkel einer vorgespannten Torsionsfeder mit mehreren Windungen angelenkt ist, wobei der zweite Federschenkel stationär am Gehäuse arretiert ist, und die Schwenkbewegung des schwenkbaren Federschenkels durch Auslösung der vorgespannten Torsionsfeder auslösbar ist, wobei in einer ersten Schwenkphase mit dem schwenkenden Federschenkel und dem an seinem Ende angelenkten Kolben-Schieber der Kolben in die Spritze schiebbar ist und damit ein Widerhaken am Kolben-Schieber an der Spritze einrastbar ist, und hernach in einer anschliessenden Schwenkbewegung des schwenkbaren Federschenkels mit dem Kolben-Schieber die Spritze im Gehäuse zurückziehbar ist.

Ein Ausführungsbeispiel dieses Selbstinjektors wird anhand der Figuren nachfolgend ausführlich beschrieben und seine Funktion wird erläutert.

Es zeigt:
- Figur 1:: Einen Selbstinjektor mit seinem unteren Gehäuseteil nach Wegnahme des oberen Gehäuseteils, mit allen darin befindlichen Komponenten, mit Aufsteckkappe auf der Nadel und zugehörigem Abzieher für das Abziehen der Aufsteckkappe;
- Figur 2:: Diesen Selbstinjektor mit allen inneren Komponenten nach Entfernung der Aufsteckkappe von der Nadel, in der Konstellation der Antriebsmittel bereit zur Injektion;
- Figur 3:: Diesen Selbstinjektor nach Figur 2 von der hinteren Seite her gesehen;
- Figur 4:: Diesen Selbstinjektor mit allen inneren Komponenten kurz nach dem Initiieren einer Injektion, mit wenig in den Spritzenzylinder eingefahrenem Kolben;
- Figur 5:: Diesen Selbstinjektor nach Figur 4 von der hinteren Seite her gesehen;
- Figur 6:: Diesen Selbstinjektor während der Injektion, nachdem der Kolben ca. die Hälfte seines Weges zurücklegte;
- Figur 7:: Diesen Selbstinjektor nach Figur 6 von der hinteren Seite her gesehen;
- Figur 8:: Diesen Selbstinjektor während der Injektion, nachdem der Kolben etwa zwei Drittel seines Weges für das Ausstossen des Wirkstoffes zurückgelegt hat;
- Figur 9:: Diesen Selbstinjektor nach Figur 8 von der hinteren Seite her gesehen;
- Figur 10:: Diesen Selbstinjektor während der Injektion, nachdem der Kolben fast seinen ganzen Weg für das Ausstossen des Wirkstoffes zurückgelegt hat;
- Figur 11:: Diesen Selbstinjektor nach Figur 10 von der hinteren Seite her gesehen;
- Figur 12:: Diesen Selbstinjektor in der Endphase der Vorwärtsbewegung des Kolbens;
- Figur 13:: Diesen Selbstinjektor nach Figur 12 von der hinteren Seite her gesehen;
- Figur 14:: Diesen Selbstinjektor am Ende der Vorwärtsbewegung des Kolbens, mit eingehängtem Widerhaken zum Zurückziehen der Spritze in einer Folgephase zur Injektion;
- Figur 15:: Diesen Selbstinjektor nach Figur 14 von der hinteren Seite her gesehen;
- Figur 16:: Diesen Selbstinjektor am Ende der Injektion;
- Figur 17:: Diesen Selbstinjektor nach Figur 16 von der hinteren Seite her gesehen;
- Figur 18:: Diesen Selbstinjektor in der Anfangsphase des Zurückziehens der Spritze;
- Figur 19:: Diesen Selbstinjektor nach Figur 18 von der hinteren Seite her gesehen;
- Figur 20:: Diesen Selbstinjektor nach vollendendem Zurückziehen der Spritze und ihrer Nadel in das Innere des Gehäuses;
- Figur 21:: Diesen Selbstinjektor nach Figur 20 von der hinteren Seite her gesehen;
- Figur 22:: Den Selbstinjektor mit geschlossenem Gehäuse;
- Figur 23:: Den Selbstinjektor nach Figur 22 mit oben geöffnetem Gehäuse, im gespannten Ausgangszustand;
- Figur 24:: Ein Detail zum Verriegelungsmechanismus des Selbstinjektors, hier gegenüber der Figur 23 in um 180° um die Längsachse gedrehter Lage, mit Blick ins Innere des oberen Gehäuseteils, und rechts darunter den kreisförmigen Ausschnitt in vergrösserter Darstellung;
- Figur 25:: einen vergrösserten Ausschnitt aus der Gehäuseoberseite von Figur 22, mit ausgeschnittenen Fenstern, um den Blick ins Innere freizugeben;
- Figur 26:: Einen Blick auf diese Fenster wie in Figur 25 gezeigt in abermals vergrösserter Darstellung, zur Unterscheidung der im Innern sichtbaren Teile;
- Figur 27:: Den Selbstinjektor in Ausgangslage, mit dem Auslöse-Schieber mit seinem Verdränger als Auslöser für eine Klinke;
- Figur 28:: Den Selbstinjektor nach Figur 27 kurz nach der Auslösung;
- Figur 29:: Den Selbstinjektor nach Figur 27 nach annähernd der Zurücklegung des halben Federschenkel-Weges;
- Figur 30:: Den Selbstinjektor nach Figur 27 am Beginn des Zurückziehens der Spritze;
- Figur 31:: Den Selbstinjektor nach Figur 27 nach Zurücklegung von ca. 2/3 des Federschenkel-Weges;
- Figur 32:: Den Selbstinjektor nach Figur 27 nach Zurücklegung von ca. 3/4 des Federschenkel-Weges;
- Figur 33:: Den Selbstinjektor nach Figur 27 nach Zurücklegung des vollen Federschenkel-Weges;
- Figur 34:: Die beiden aufeinander zu klickenden Gehäuseteile des Selbstinjektors zur Illustration des Auslösemechanismus mit dem Verdränger für eine Klinke;
- Figur 35:: Eine Sicherungsplatte zum Draufsetzen des Selbstinjektors für die Montage.

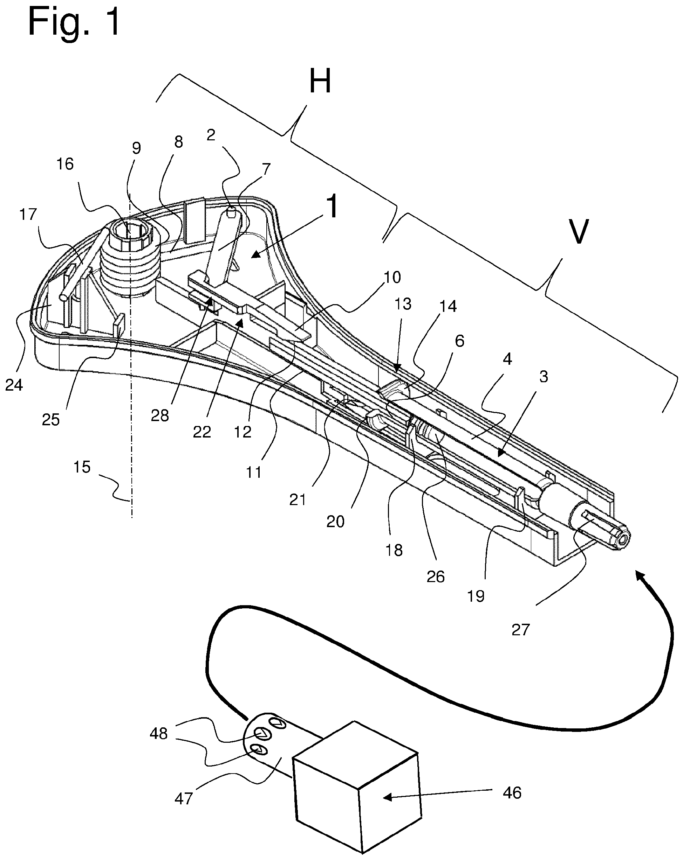

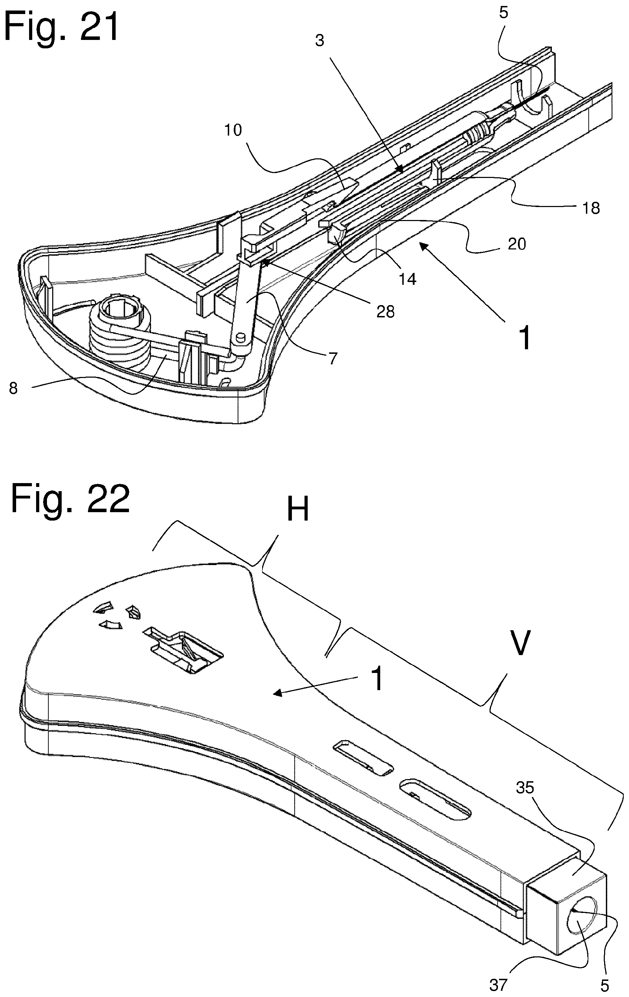

Die **Figur 1** zeigt den Selbstinjektor mit seinem unteren Gehäuseteil 1 nach Wegnahme des oberen Gehäuseteils, mit allen darin befindlichen Komponenten, mit einer Aufsteckkappe 27 auf der Nadel, die hier deswegen nicht sichtbar ist. Im Folgenden werden zunächst alle Teile bzw. Komponenten dieses Selbstinjektors bezeichnet und beschrieben. Im hinteren Gehäuseteil H ist die ganze Antriebsmechanik für den Selbstinjektor untergebracht. Zuhinterst im Gehäuse 1 befindet sich ein Bolzen 16 in Form eines aus Kunststoff gespritzten Hohlzylinders, auf dem eine Torsionsfeder 9 lagert. Die Achse 15 der Torsionsfeder 9 ist eingezeichnet und verläuft senkrecht zum Gehäuseboden. Ein Federschenkel 17 dieser Torsionsfeder 9 ist stationär gelagert und schlägt am Gehäuse 1 an und ist hierzu in einem entsprechend ausgeformten Rückhalter 24 festgehalten. Der andere Federschenkel 8 ist beweglich und ist mit seinem äusseren Ende mit dem hinteren Ende eines Schwenkhebels 7 über das Kniegelenk 2 verbunden. Der bewegliche Federschenkel 8 kann kraft der Torsionsfeder 9 von dieser gezeigten Ausgangsposition im Bild im Uhrzeigersinn schwenken, bis er am Begrenzungsnocken 25 anschlägt. Damit schwenkt er den Schwenkhebel 7 ebenfalls von der hier gezeigten Ausgangsposition in Bezug auf die Längsachse des Gehäuses 1 in eine etwa symmetrisch gegenüberliegende Lage. Gleichzeitig stösst der Schwenkhebel 7 damit den mit ihm über ein Gelenk 28 verbundenen Kolben-Schieber 22 von hinten nach vorne, also in Richtung wo sich die Spritze 3 befindet, bis der Schwenkhebel 7 in der gleichen Richtung wie der Kolben-Schieber 22 verläuft, und beim weiteren Schwenken zieht er den Kolben-Schieber 22 ein stückweit zurück, bis der bewegliche Federschenkel 8 am Begrenzungsnocken 25 anschlägt. Vor dem Gebrauch ragt die Nadel der Spritze vorne aus dem Gehäuse 1 und ist mit einer Gummi- oder Kunststoff-Aufsteckkappe 27 geschützt, weswegen sie hier nicht sichtbar ist. Sie ist von einer kubischen oder quaderförmigen Schutzkammer 35 mit vorne einem Loch 37 vollständig eingeschlossen, wobei diese Schutzkammer erst ab Figur 22 gezeigt ist. Sie dient einerseits als Schutz für die Nadel und andererseits für das Auslösen des Selbstinjektors, wie das später noch klar wird. Unten ist noch ein Abzieher 46 gezeigt. Auf die Aufsteckkappe 27 und über die Schutzkammer 35 ist ein solcher Abzieher 46 für die Aufsteckkappe 27 aufgesetzt, ein etwa kubischer oder quaderförmiger Kunststoff-Hohlkörper mit einer offenen Seite, aus welchem ein Metallrohrabschnitt 47 mit nach innen ragenden Widerhaken 48 ragt. Dieser Abzieher 46 wird mit seinem Rohrabschnitt 47 von vorne über die Aufsteckkappe 27 gestülpt, sodass also der Selbstinjektor mit eingesetzter Spritze mit diesem kubischen Abzieher 46 bestückt ausgeliefert wird und er schliesst im Lieferzustand direkt an die hier noch nicht gezeigte Schutzkammer 35 an. Das Gehäuse endet daher vorne mit diesem kubischen oder quaderförmigen geschlossenen Ende, nämlich mit diesem Abzieher 46. Für den Einsatz der Spritze 3 braucht der Anwender nur die Aufsteckkappe 27 zu entfernen, indem er diesen Abzieher 46 vom Gehäuse 1 wegzieht, und dessen Widerhaken 48 ziehen die Aufsteckkappe 27 sicher von der Nadel ab. Auch dann ist die Nadel aber noch im Innern einer Schutzkammer 35 mit vorne einem Loch 37 geschützt. Diese Schutzkappe 35 gehört zum Auslösemechanismus und wird ab der Figur 22 gezeigt und beschrieben. Der Benützer kann diese Schutzkammer 35 mit ihrem Loch 37 direkt an der gewünschten Stelle an seinen Körper pressen. Beim Aufdrücken des Selbstinjektors auf die Haut wird die Schutzkammer 35 ein stückweit in das Gehäuse 1 geschoben und löst damit die Injektion aus. Es erfolgt dann automatisch das Injizieren des Wirkstoffes und hernach erfolgt sofort und automatisch das Zurückziehen der Nadel in das Gehäuse 1 hinein, sodass sie darin komplett verschwindet und niemanden mehr verletzen oder infizieren kann.

Der Kolben-Schieber 22 ist am vorderen Ende einstückig ausgestaltet in Form eines Kolbens 6, der eintaucht in das offenen Ende des Zylinders 4 der Spritze 3, die in den vorderen Gehäuseteil V eingelegt ist, und dieser Kolben-Schieber 22 ist längs einer Schiene 11, die eine Nut bildet, am Gehäuse 1 längsverschiebbar geführt. Die Spritze 3 liegt auf einer Halterung im Gehäuse 1, hier in Form von zwei oben U-förmig ausgeformten Stegen 18, 19, in welche die Spritze 3 mit ihrem Zylinder 4 einpasst. Vorne am Kolben 6 erkennt man einen Dichtzapfen 26, der sich im Innern des Zylinders 4 dichtend nach vorne schieben lässt, zum hydraulischen Auspressen des Spritzeninhaltes vorne aus der Nadel. Am Kolben-Schieber 22 ist ein elastischer Widerhaken 10 angeformt, mit auf seiner Unterseite einer Aufschieberampe 12, mit der er über das hintere Ende 13 der Spritze 3 fahren kann und dann am Flansch 14 der Spritze 3 einhakt, um sie nach erfolgter Injektion in das Gehäuse 1 zurückzuziehen. Damit die Spritze 3 beim Einstechen der Nadel aber nicht im Gehäuse 1 nach hinten geschoben werden kann, ist ein Anschlag 20 für den Flansch 14 im Gehäuseboden 1 ausgeformt. Hinten anschliessend an diesen Anschlag 20 befindet sich eine schräg nach hinten abfallende bzw. schräg nach vorne ansteigende Rampe 21. Wird der Kolben-Schieber 22 nach vorne verschoben, so fährt er am Schluss mit seinem unteren Rand, der ebenso eine hier nicht sichtbare Rampe ausformt, auf diese Rampe 21 auf und drückt den elastischen Schenkel 29 nach unten, wodurch der Anschlag 20 den Flansch 14 freigibt, damit die Spritze 3 ungehindert vom Widerhaken 10 in das Gehäuse gezogen werden kann.

Im Folgenden wird die Funktion dieses Selbst-Injektors Schritt für Schritt dargestellt, beschrieben und erklärt, und zwar anhand von chronologischen Bildfolgen, welche den unteren Gehäuseteil und die inneren Teile zunächst bis auf den Auslösemechanismus zeigen.

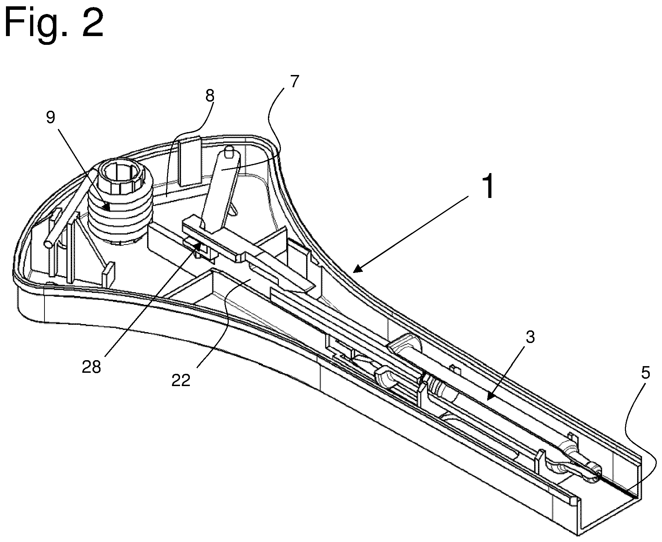

Hierzu zeigt die **Figur 2** den Selbstinjektor nach Entfernung der Aufsteckkappe 27 sodass nun die Nadel 5 sichtbar ist. In dieser Konstellation des Antriebsmechanismus ist der Selbstinjektor bereit für die Injektion des in der Spritze 3 enthaltenen Wirkstoffes. Die Torsionsfeder 9 ist gespannt, das heisst ihr beweglicher Federschenkel 8 befindet sich in der hier gezeigten, vorgespannten Position und ist in derselben gesichert, weil der Kolben-Schieber 22 von einer auslösbaren Klinke arretiert ist, wie dieser Auslösemechanismus zu den Figuren 22 ff. noch im Einzelnen beschrieben wird. Für die Anwendung drückt der Benützer die hier nicht gezeigte Schutzkammer 35 an der gewünschten Stelle auf den Körper. Sie wird ein stückweit ins Gehäuse 1 geschoben und die Nadel 5 dringt in die Haut ein und gleichzeitig wird die Injektion ausgelöst, wie noch beschrieben wird. Der bewegliche Federschenkel 8 startet nach der Auslösung seine Schwenkbewegung, was die Injektion bewirkt. Anschliessend wird die Spritze 3 mitsamt der Nadel 5 automatisch ins Gehäuse zurückgezogen, sodass die Nadel 5 im Gehäuse 1 verschwindet und niemanden verletzen oder infizieren kann.

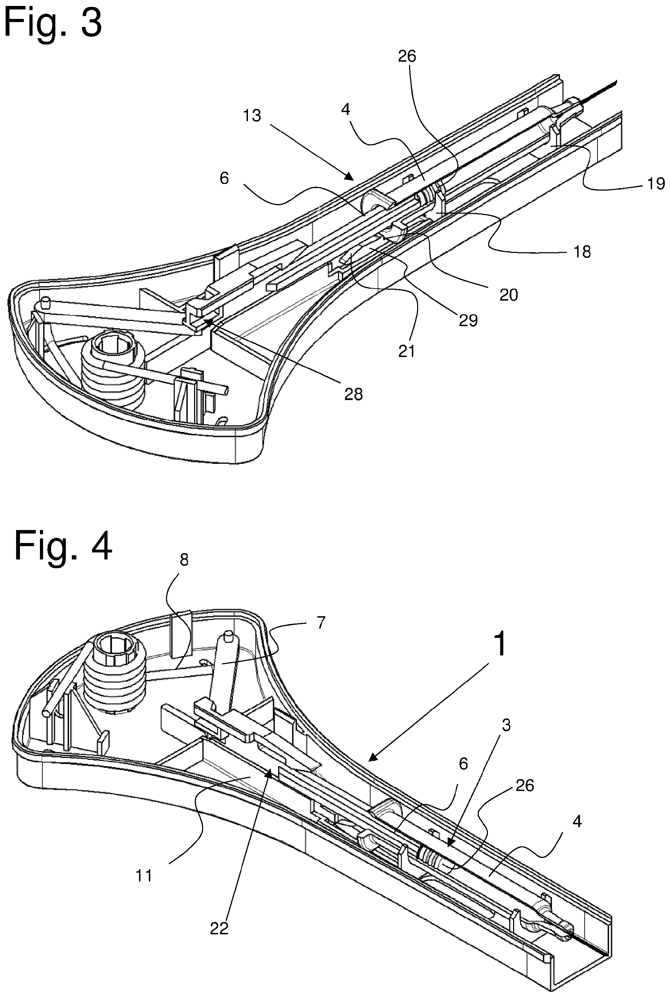

Die **Figur 3** zeigt diesen Selbstinjektor in der Position wie in **Figur 2** gezeigt von der anderen, hinteren Seite her gesehen. Man sieht hier die Halterungen für die Spritze, nämlich die oben U-förmig ausgenommenen Stege 18, 19, auf welchen die eingelegte Spritze 3 mit ihrem Zylinder 4 aufliegt. Weiter sieht man den Anschlag 20, welcher ein weiteres Rückwärtsverschieben der Spritze 3 in das Gehäuse 1 hinein verhindert, und die Auffahrrampe 21, die an einem Schenkel 29 ausgeformt ist, der im Gehäuseboden wurzelt, und auf welche der Kolben-Schieber 22 auffahrbar ist, zum Freigeben des hinteren Spritzenendes 13.

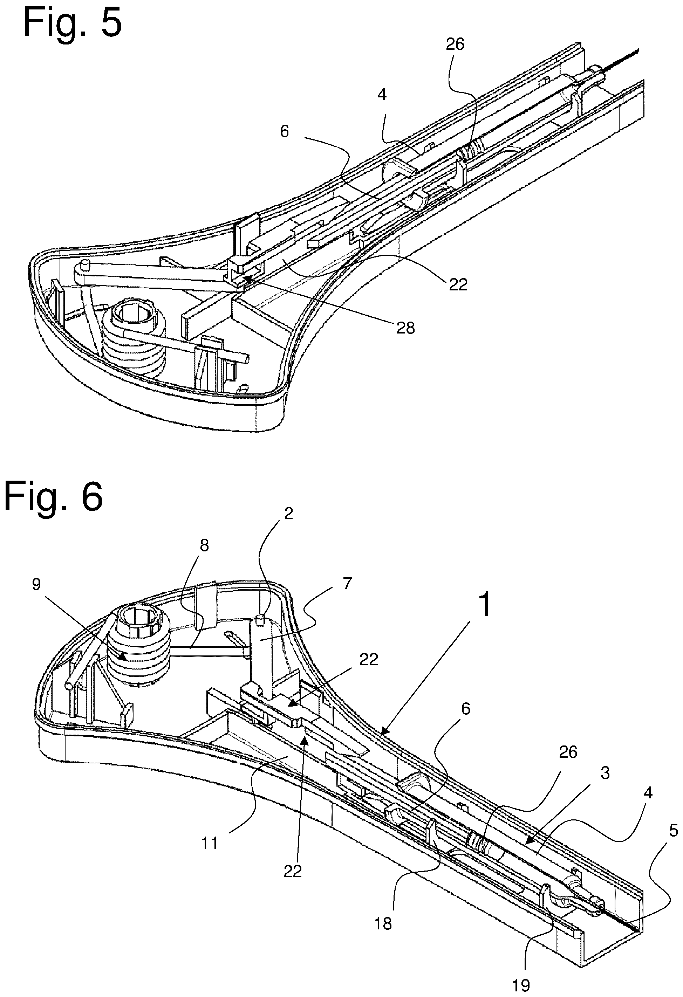

In **Figur 4** ist der Selbstinjektor kurz nach dem Initiieren einer Injektion gezeigt, mit bereits geringfügig in den Spritzenzylinder 4 eingefahrenem Kolben 6. Wie man sieht hat der bewegliche Federschenkel 8 ein Stück Weg zurückgelegt und damit den Hebel 7, der mit seinem vorderen Ende über das Gelenk 28 am Kolben-Schieber 22 angelenkt ist, längs der Schiene 11 für den Kolben-Schieber 22 nach vorne geschoben. Mit seinem vorderen Ende ist der Kolben 6 in den Zylinder 4 der Spritze 3 eingefahren und drückt den Dichtzapfen 26 darin nach vorne. Entsprechend wird Wirkstoff aus dem Inneren des Zylinders 4 durch die Nadel 5 gespritzt. Die **Figur 5** zeigt den Selbstinjektor in der Position wie in **Figur 4** gezeigt von der anderen, hinteren Seite her gesehen. Vor dem vorderen Ende des Kolbens 6 im Zylinder 4 der Spritze 3 liegt der Dichtzapfen 26.

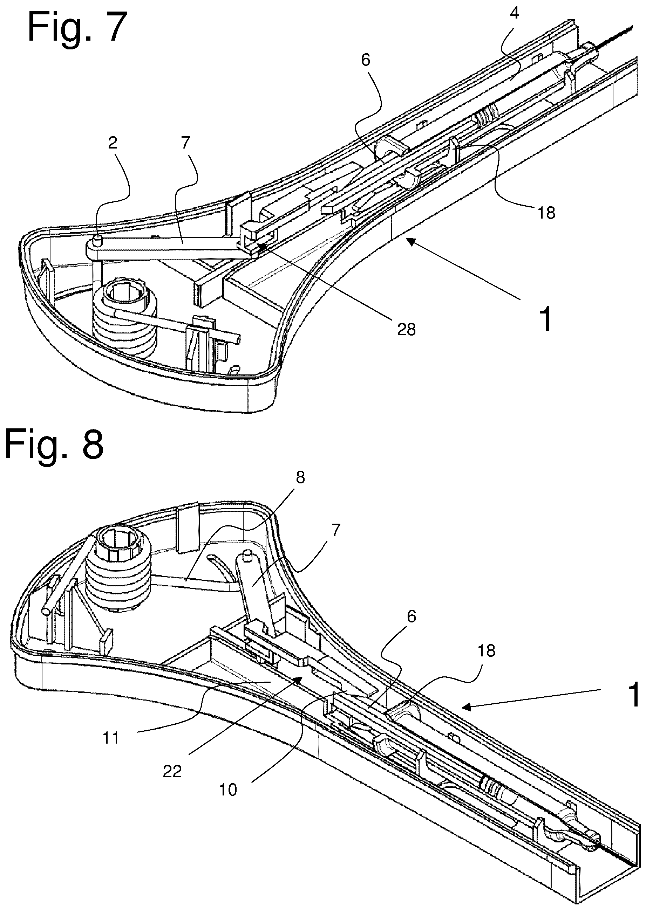

In **Figur 6** ist der Selbstinjektor im weiteren Verlauf der Injektion gezeigt, nachdem der Kolben 6 ca. die Hälfte seines Weges zurücklegte. Der Dichtzapfen 26 befindet sich nun etwa in der Mittel des Zylinders 4 der Spritze 3. Der bewegliche Federschenkel 8 schwenkte weiter und auch der an ihm angelenkte Schwenkhebel 7. Wie man sieht bildet der Federschenkel 8 zusammen mit dem Schwenkhebel 7 ein Kniegelenk 2. Die vom Federschenkel 8 in den Schwenkhebel 7 eingeleitete Kraft nimmt naturgemäss mit forschreitender Schwenkbewegung des Federschenkels 8 ab. Gleichzeitig kann auf den Kolben-Schieber 22 umso mehr Stosskraft aufgebracht werden, je gestreckter dieses Kniegelenk 2 ist. Das ist wesentlich, denn dadurch bleibt die auf den Dichtzapfen 26 wirkende Ausstosskraft während der ganzen Injektion gleichmässig Die **Figur 7** zeigt den Selbstinjektor in der Position wie in **Figur 6** gezeigt von der anderen, hinteren Seite her gesehen.

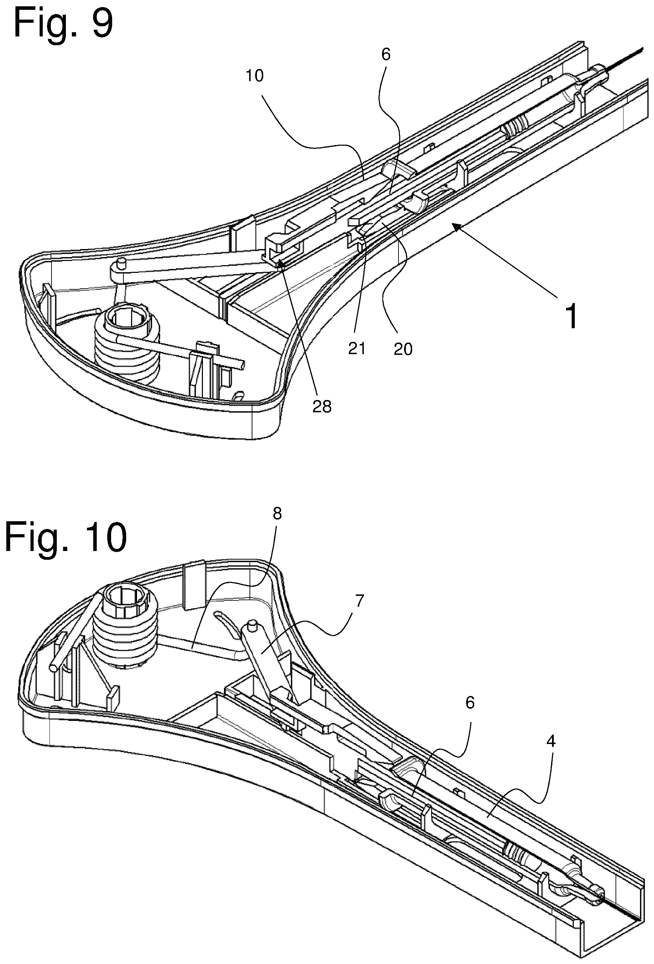

Die **Figur 8** zeigt den Selbstinjektor während der Injektion, nachdem der Kolben 6 etwa zwei Drittel seines Weges für das Ausstossen des Wirkstoffes zurückgelegt hat. Entsprechend ist der bewegliche Federschenkel 8 weitergeschwenkt und hat den an ihn angelenkten Schwenkhebel 7 ebenfalls weitergeschwenkt, sodass dessen vorderes Ende den Kolben-Schieber 22 längs der Schiene 11 weiter nach vorne schob. Die **Figur 9** zeigt den Selbstinjektor in der Position wie in **Figur 8** gezeigt von der anderen, hinteren Seite her gesehen.

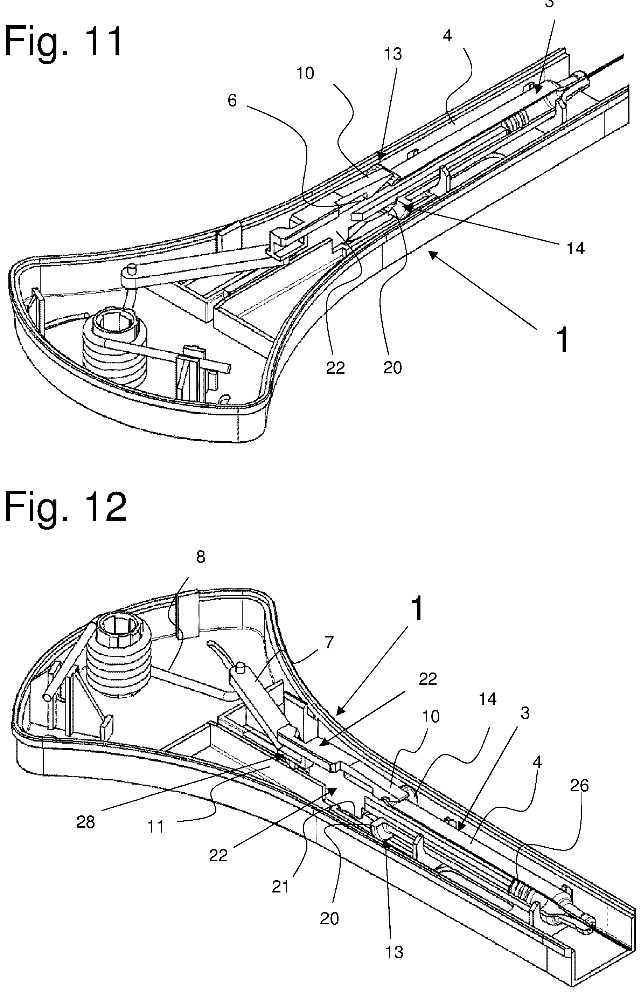

In der **Figur 10** ist der Selbstinjektor während der Injektion gezeigt, nachdem der Kolben 6 im Zylinder 4 fast seinen ganzen Weg für das Ausstossen des Wirkstoffes zurückgelegt hat, und in **Figur 11** ist diese Position von der anderen, hinteren Seite des Selbstinjektors her gesehen gezeigt. Der Widerhaken 10 am Kolben-Schieber 22 steht in Bezug auf das hintere Ende der Spritze 3 kurz vor seinem Auffahren auf den Flansch 14 am hinteren Ende 13 der Spritze 3, um an diesem Flansch 14 einzurasten.

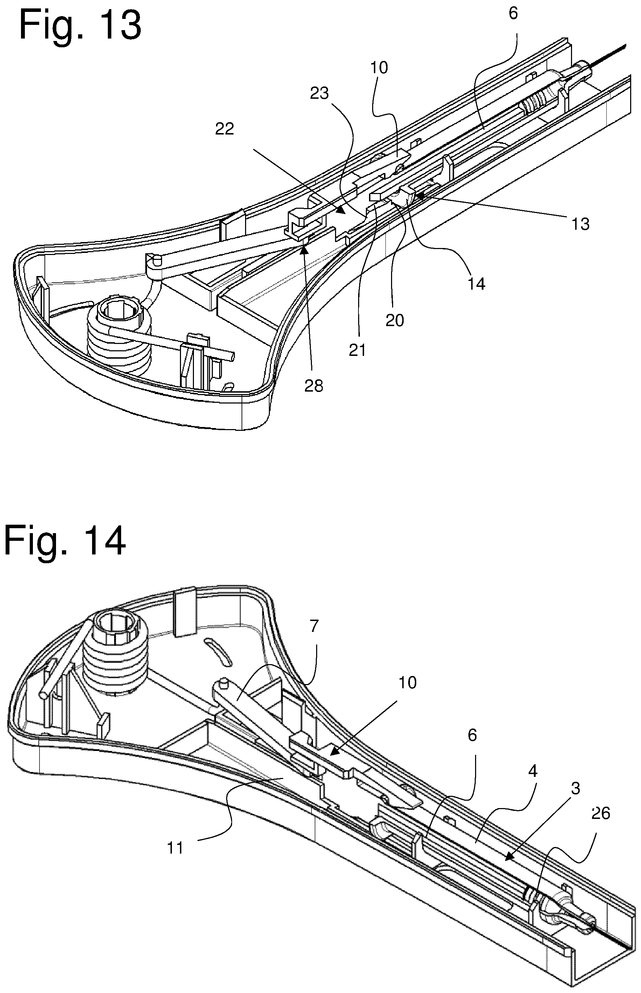

Die **Figur 12** zeigt den Selbstinjektor in der Endphase der Kolbenbewegung. Der Dichtzapfen 26 in der Spritze 3 bzw. in ihrem Zylinder 4 ist fast am vorderen Ende angelangt. Und der Schwenkhebel 7 für das Vorwärtsschieben des Kolben-Schieberse 22 ist nur noch leicht von der Verlaufrichtung der Schiene 11 abgewinkelt. Wie man sieht ist der Widerhaken 10 hier schon zur Hälfte auf den Flansch 14 am hinteren Ende der Spritze 3 aufgefahren. Gleichzeitig ist der Kolben-Schieber 22 schon ein stückweit auf der schräg ansteigenden Rampe 21 aufgefahren, um den elastischen Schenkel 29 nach unten zu drücken, wodurch der Anschlag 20 den Flansch 14 freigibt, damit die Spritze 3 ungehindert vom Widerhaken 10 nach hinten gezogen werden kann. Die **Figur 13** zeigt dieselbe Situation von der anderen, hinteren Seite des Selbstinjektors her gesehen.

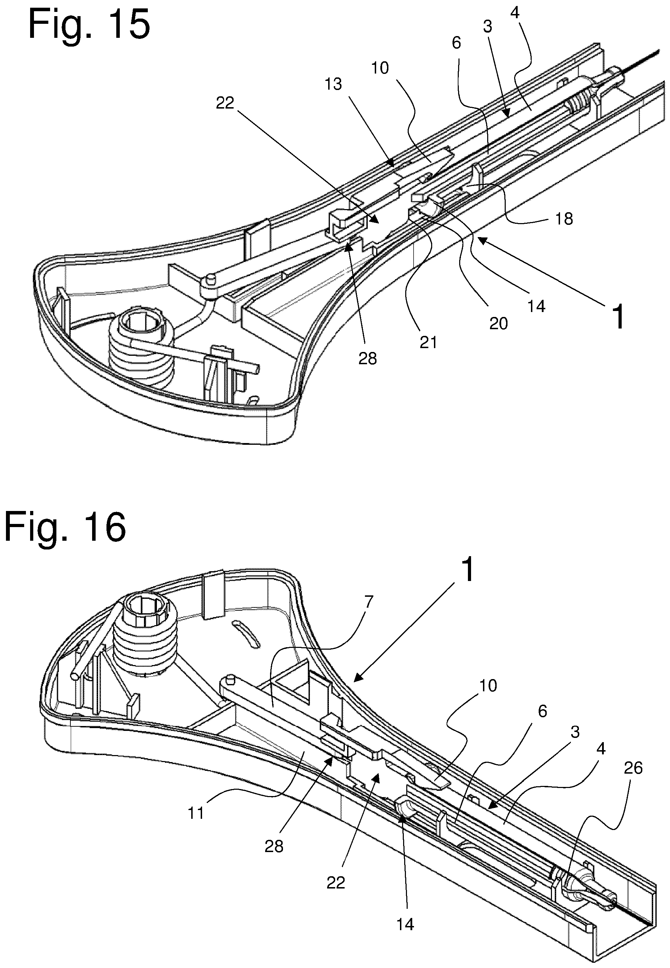

Die **Figur 14** zeigt den Selbstinjektor kurz vor dem Ende der Kolbenbewegung. Der Schwenkhebel 7 ist jetzt fast genau auf die Schiene 11 ausgerichtet und der Kolben 6 entsprechend fast maximal nach vorne in den Zylinder 4 der Spritze 3 hineingeschoben. Die weitere **Figur 15** zeigt dasselbe von der anderen, hinteren Seite des Selbstinjektor her gesehen, mit dem auf den Flansch 14 am Ende 13 der Spritze 3 aufgefahrenen Widerhaken 10 nach seinem Einhaken an diesem Flansch 14. Der Widerhaken 10 wird hierzu gegenüber dem Kolben-Schieber 22 leicht elastisch emporgeschenkt, und fällt dann wie eine Klinke hinter dem Flansch 14 aufgrund der elastischen Rückstellkraft wieder nieder und rastet am Flansch 14 ein. Der Kolben-Schieber 22 ist auf die aufsteigende Rampe 21 aufgefahren und hat den elastischen Schenkel 29 und damit den Anschlag 20 nach unten gedrückt, sodass hernach die Spritze 3 ungehindert ins Gehäuse 1 zurückgezogen werden kann.

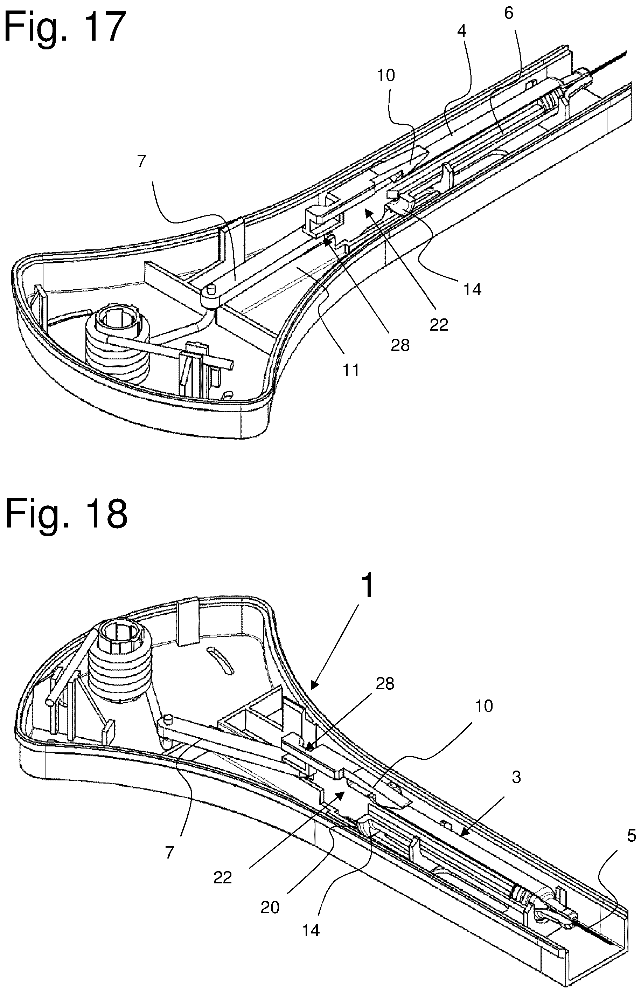

In der Darstellung nach der **Figur 16** ist Selbstinjektor nach Vollenden der Injektionsphase gezeigt. Der Widerhaken 10 ist jetzt am Flansch 14 der Spritze 3 eingehängt. Der Kolben 6 ist mit dem Dichtzapfen 26 am vorderen Ende des Zylinders 4 angekommen. Der Schwenkhebel 7 befindet sich nun etwa in der gleichen Geraden wie die Führungsschiene 11 für den Kolben-Schieber 22 und den Kolben 6. Weil der Widerhaken 10 am Flansch 14 eingerastet ist, ist damit die Spritze 3 bereit, um vom Kolben-Schieber 22 und dem Widerhaken 10 in der anschliessenden Phase zurückgezogen zu werden. Die **Figur 17** zeigt diese Position von der anderen, hinteren Seite des Selbstinjektors her gesehen.

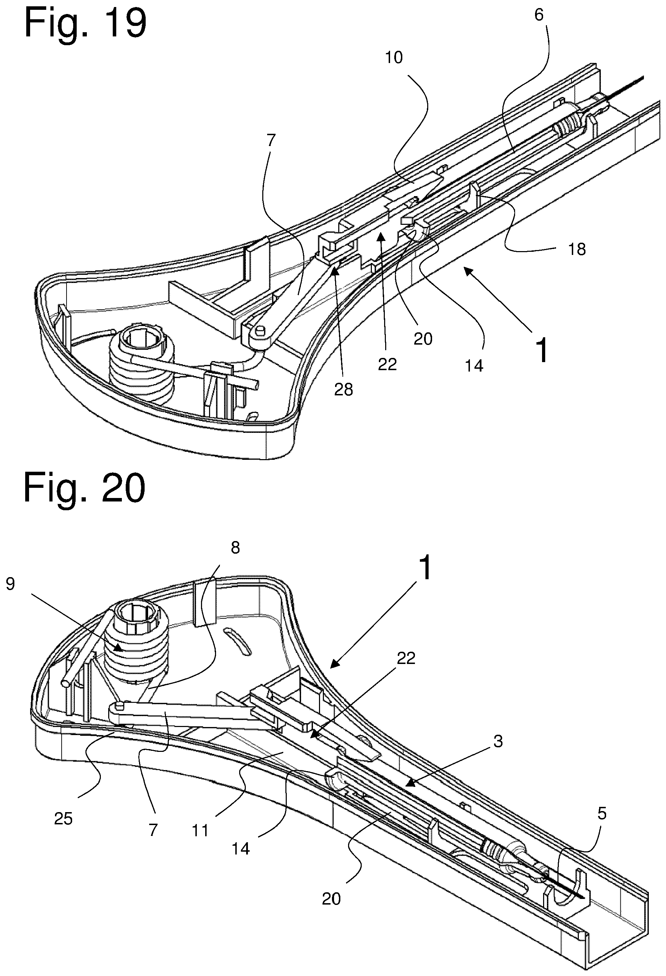

In **Figur 18** ist die Anfangsphase des Zurückziehens der Spritze 3 dargestellt. Der Schwenkhebel 7 ist hier in seiner Schwenkung über die Verlaufrichtung der Schiene 11 hinaus bereits weitergeschwenkt und zieht mit seiner vorderen Anlenkung 28 am Kolben-Schieber 22 denselben in das Gehäuse 1 hinein. Der Widerhaken 10 an am Kolben-Schieber 22 zieht daher die Spritze 3 an ihrem hinteren Flansch 14 zurück und damit auch die Nadel 5 in das Gehäuse 1 hinein. Die **Figur 19** zeigt dieselbe Position des Schwenkhebels 7 von der anderen, hinteren Seite des Selbstinjektors her gesehen. Auch hier sieht man wie die Spritze 3 mit ihrem Flansch 14 ungehindert am nach unten gedrückten elastischen Schenkel 29 und Anschlag 20 vorbei vom Widerhaken 10 und dem Kolben-Schieber 22 zurückgezogen werden kann.

Am Schluss der ganzen Aktion des Schwenkhebels 7 mittels der Torsionsfeder 9 stellt sich die Situation wie in **Figur 20** dargestellt ein. Der bewegliche Federschenkel 8 ist an seinem Anschlag 25 im Gehäuse 1 angekommen und hat den Schwenkhebel 7 bis dorthin mitgeschwenkt. Weil das vordere Ende des Schwenkhebels 7 am Kolben-Schieber 22 angelenkt ist, hat der Schwenkhebel 7 den Kolben-Schieber 22 zurückgezogen und mit ihm über den Widerhaken 10 auch die ganze Spritze 3, sodass ihre Nadel 5 letztlich gänzlich in das Gehäuse 1 hineingezogen wurde und niemand mehr verletzen oder infizieren kann. Schliesslich zeigt die **Figur 21** diese Situation noch von der anderen, hinteren Seite des Selbstinjektors her gesehen. Die Spritze 3 wurde über den Anschlag 20 hinweg vom Widerhaken 10 am Flansch 14 nach hinten gezogen. Die Nadel 5 der Spritze 3 ist im Gehäuse 1 verschwunden.

Es versteht sich, dass auf den unteren Gehäuseteil, der in diesen Figuren 1 bis 21 gezeigt wurde, ein deckungsgleicher oberer Gehäuseteil gehört, der auf den unteren aufklickbar ist, und sich ein geschlossenes Gehäuse 1 bildet, sodass sich der Selbstinjektor dann wie in Figur 22 gezeigt darstellt.

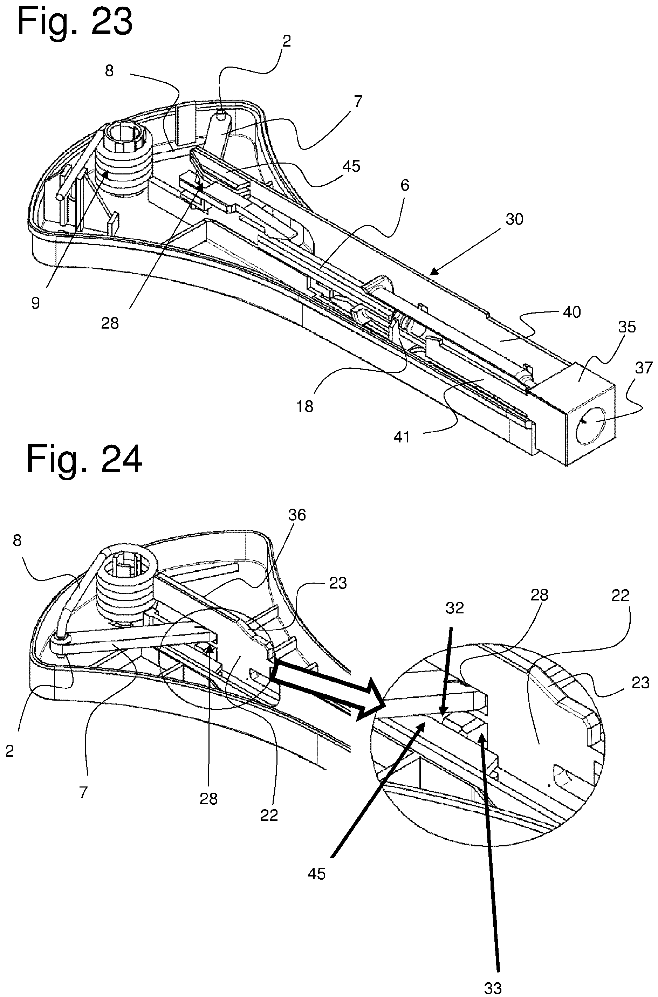

Das Gehäuse 1 dieses Selbstinjektors formt in seinem vorderen Teil V, in welchen die Spritze 3 eingelegt wird, einen im Querschnitt rechteckigen Kanal, in dem der Kolben-Schieber 22 auch mit seiner Oberseite geführt ist, das heisst auch an der Innenseite des oberen Gehäuseteils. Der hintere Teil H des Gehäuses 1 weitet sich von diesem rechteckigen Kanal nach hinten beidseits fächerartig auf. Die Nadel 5, die Torsionsfeder 9 und das Röhrchen 47 am Abzieher 46 (Figur 1) sind die einzigen Teile aus Metall an einem mit einer Spritze geladenen Selbst-Injektor. Alle anderen Teile dieses Selbstinjektors können aus Spritzkunstoff gefertigt werden, und wenn es sein muss kann auch der Abzieher aus Kunststoff hergestellt werden. Der Zusammenbau ist einfach, denn es gibt nur wenige Teile zu montieren, nämlich nebst der Spritze 3 aus Zylinder 4, Dichtzapfen 26, eingeschlossenem Wirkstoff und Nadel 5 mit Schutzkappe 27 bloss sieben Teile, nämlich:
1. Unterer Gehäuseteil
2. Oberer Gehäuseteil
3. Torsionsfeder 9
4. Schwenkhebel 8
5. Kolben-Schieber 22 mit einstückig daran ausgeformtem Kolben 6 und Widerhaken 10
6. Auslöse-Schieber 30 zum Auslösen, mit Auslöseverdränger 45 (ab Figur 23)
7. Abzieher 46 mit Röhrchen 47 und Widerhaken 48 darin, wie in Figur 1 gezeigt Der Auslöse-Schieber 30 mit seinem Auslöseverdränger 45 und dessen Funktion wird anhand der Figuren 23-34 noch genauer beschrieben.

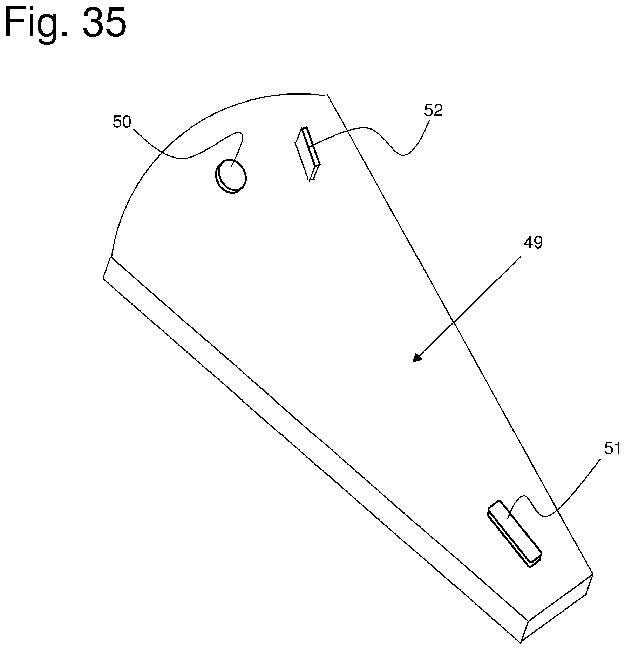

Die Montage geht wie folgt vonstatten. Das untere Gehäuseteil wird auf einer Montagehilfe 49 positioniert, wie sie in Figur 35 gezeigt wird. Die Torsionsfeder 9 wird über den Bolzen 16 im Gehäuse 1 gesteckt, derart, dass der Federschenkel 17 stationär gelagert ist und am Gehäuse 1 in einem entsprechend ausgeformten Rückhalter 24 anschlägt und festgehalten wird, und dass der Federschenkel 8 unter Kraftaufwendung vorgespannt wird in seine Ausgangsposition und da vorübergehend blockiert wird in seiner Position, beispielsweise mittels eines Nockens 52 auf der Montageplatte 49. Der Schwenkhebel 7 wird im Gelenk 28 am hinteren Ende des Kolben-Schiebers 22 eingeclipst. Die so vormontierten Teile Schwenkhebel 7 und Kolben-Schieber 22 werden nun in die Schiene 11 im unteren Gehäuseteil eingesetzt. Zugleich wird der Schwenkhebel 7 mit seinem hinteren Ende mit dem Ende des beweglichen Federschenkels 8 über den Anlenkpunt 2 verbunden. Der Abzieher 46 mit seinem Röhrchen 47 mit seinen Widerhaken 48 wird auf die Schutzkammer 35 des Auslöse-Schiebers 30 gesteckt. Nun wird vom hinteren Ende des Auslöse-Schiebers 30 mit aufgestecktem Abzieher 46 die Spritze 3 bestehend aus Zylinder 4, Dichtzapfen 26, eingeschlossenem Wirkstoff und Nadel 5 mit Schutzkappe 27 durchgesteckt, so dass die Schutzkappe 27 durch das Loch 37 der Schutzkammer 35 durchtaucht und sich mit den Widerhaken 48 verkrallt. Die so vormontierten Teile Auslöse-Schieber 30, Spritze 3 und Abzieher 46 werden in das untere Gehäuseteil eingelegt, so dass der Kolben 6 am vorderen Ende des Kolben-Schiebers 22 in das offene Ende des Zylinders 4 der Spritze 3 eintaucht, ohne dabei den Dichtzapfen 26 zu berühren. Jetzt wird das obere Gehäuseteil auf das untere Gehäuseteil schlüssig aufgesetzt. Dabei taucht die Auslöseklinke 33 des oberen Gehäuseteils ein in die Ausnehmung 32 im Kolben-Schieber 22. Das montierte Gehäuse kann nun von der Montageplatte 49 entfernt werden. Dadurch gibt der Nocken 52 den vorgespannten Federschenkel 8 frei. Der Kolben-Schieber 22 wird jedoch von der Auslöseklinke 33
durch einen Auslöse-Schieber 30 zunächst zurückgehalten, sodass der Federschenkel 8 gespannt bleibt.

Diese Auslöseklinke und das Auslösen des Selbst-Injektors wird im Detail anhand der nachfolgenden Figuren beschrieben.

Die Figuren 22 bis 32 zeigen hierzu den Selbstinjektor mit jetzt eingesetztem Auslöse-Schieber 30. Zunächst wurde dieser Selbstinjektor in **Figur 22** mit geschlossenem Gehäuse dargestellt. In Figur 23 ist der obere Gehäuseteil wieder entfernt, und wie man sieht befindet sich der Selbstinjektor hier in der gespannten Ausgangslage, also mit gespannter Torsionsfeder 9. Der bewegliche Federschenkel 8 wird in diesem gespannten Zustand zurückgehalten, weil der Kolben-Schieber 22 von einer Klinke blockiert und zurückgehalten wird. Hier ist nun erstmal der Auslöse-Schieber 30 eingesetzt, der an seinem hinteren Ende einen Verdränger 45 ausformt, und mit einer Schieberplatte 40 am Gehäuse geführt ist. Auf der gegenüberliegenden Seite der Schieberplatte 40 ist eine zweite Führungsplatte 41 an der Schutzkammer 35 angeformt, die auch längs des Gehäuses verschiebbar ist.

Die **Figur 24** zeigt ein Detail zum Verriegelungsmechanismus des Selbstinjektors. Die Darstellung zeigt die Konstruktion in einer gegenüber der Darstellung in Figur 23 um 180° um die Längsachse des Gehäuses 1 gedrehten Lage, ohne den bisher gezeigten unteren Gehäuseteil, aber mit dem oberen Gehäuseteil mit Blick in sein Inneres, und rechts darunter den kreisförmigen Ausschnitt in einer vergrösserten Darstellung. Hier erkennt man den Kolben-Schieber 22, dessen unterer Rand 36 in dieser Figur oben liegt, eine Schrägfläche oder Rampe 23, mit welcher der Kolben-Schieber 22 auf die Rampe 21 auffahren kann, um den elastischen Schenkel 29 und damit den Anschlag 20 wegzudrücken, sodass hernach die Spritze 3 ungehindert ins Gehäuse 1 zurückgezogen werden kann

In der vergrösserten Darstellung der Verriegelung sieht man die im Gehäuse seitlich ausschwenkbare Klinke 33, und den jetzt seitlich an ihr anliegenden Verdränger 45, der die Klinke 33 in einer Ausnehmung 32 im Kolben-Schieber 22 hält, sodass der Kolbenschieber 22 sich nicht in Richtung Spritze 3 verschieben kann.

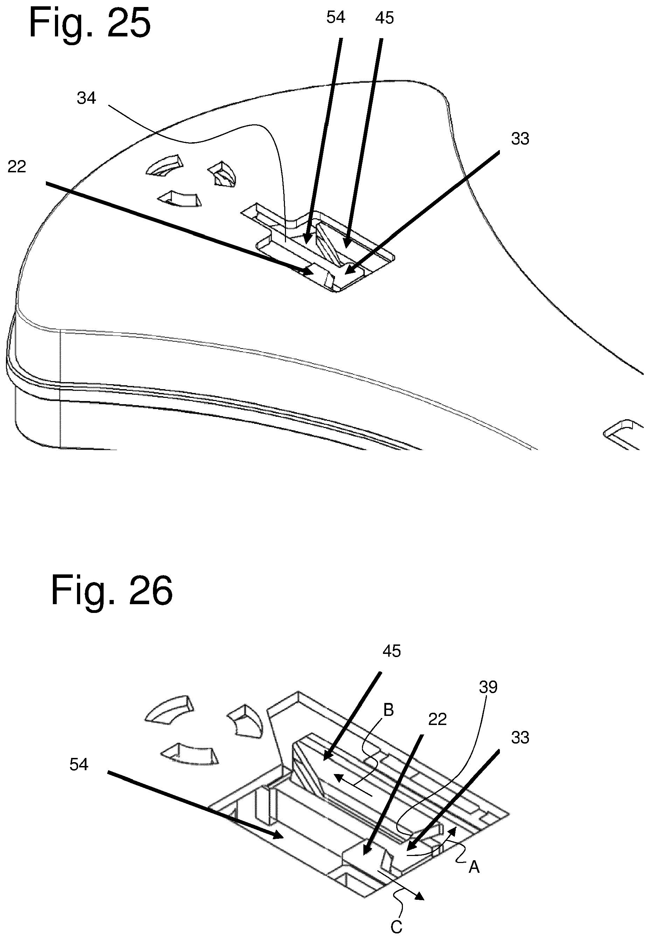

Die Abfolge des Bewegungsablauf ab der Auslösung dieses Selbstinjektors erschliesst anhand der folgenden Beschreibung. Die **Figur 25** zeigt den hinteren Teil des oberen Gehäuseteils mit einem Fenster 44, welche den Blick ins Innere freigibt. In diesem Fenster 44 erkennt man eine Klinke 33 am Ende eines elastisch schwenkbaren Arms 34, der am Gehäuseteil angeformt ist. Diese Klinke 33 kann quer zu diesem Arm elastisch hin und her schwenken. An den hier im Bild hinteren äusseren Rand der Klinke 33 liegt der Verdränger 45 des Auslöse-Schiebers 30 an und drückt damit die Klinke 33 gegen ihre elastisch Rückstellkraft in die Ausnehmung 32 im Kolben-Schieber 22. Damit ist der Kolben-Schieber 22 blockiert und kann nicht in Richtung zur Spritze 3 hin verschoben werden. Der Federschenkel 8 und auch der Schwenkarm 7 sind entsprechend ebenfalls an einer Schwenkung gehindert.

In der **Figur 26** ist mit einer abermals vergrösserten Darstellung des Fensters bzw. der Ausnehmung 44 im oberen Gehäuseteil gezeigt, wie durch das Applizieren des Selbstinjektors, also durch Aufdrücken der Schutzkammer 35 auf die geplante Einstichstelle der Auslöse-Schiebers 30 nach hinten ins Gehäuse geschoben wurde, gemäss Pfeil B. Damit wurde der Verdränger 45 am Auslöse-Schieber 30 an dieser Klinke 33 gleitend vorbeigeschoben. Dieses Vorbeischieben geschieht, bis die hintere Kante 39 des Auslösers 45 die Klinke 33 passiert und sie für eine Schwenkung hin gegen den Auslöse-Schieber 30 freigibt. Damit schwenkt die Klinke 33 an ihrem Arm 34 aus der Ausnehmung 32 am Kolben-Schieber 22 heraus, gemäss PfeilA, und gibt ihn für eine Verschiebung Richtung Spritze 3 schlagartig frei. Der Kolben-Schieber 22, angetrieben vom vorgespannten Federschenkel 8 und dem daran angelenkten Schwenkhebel 7, mit dem er zusammen einen Kniehebel bildet, schiebt sodann gemäss Pfeil C den Kolben 6 innerhalb der Spritze 3 nach vorne und der Wirkstoff wird durch die Nadel ins Gewebe gespritzt.

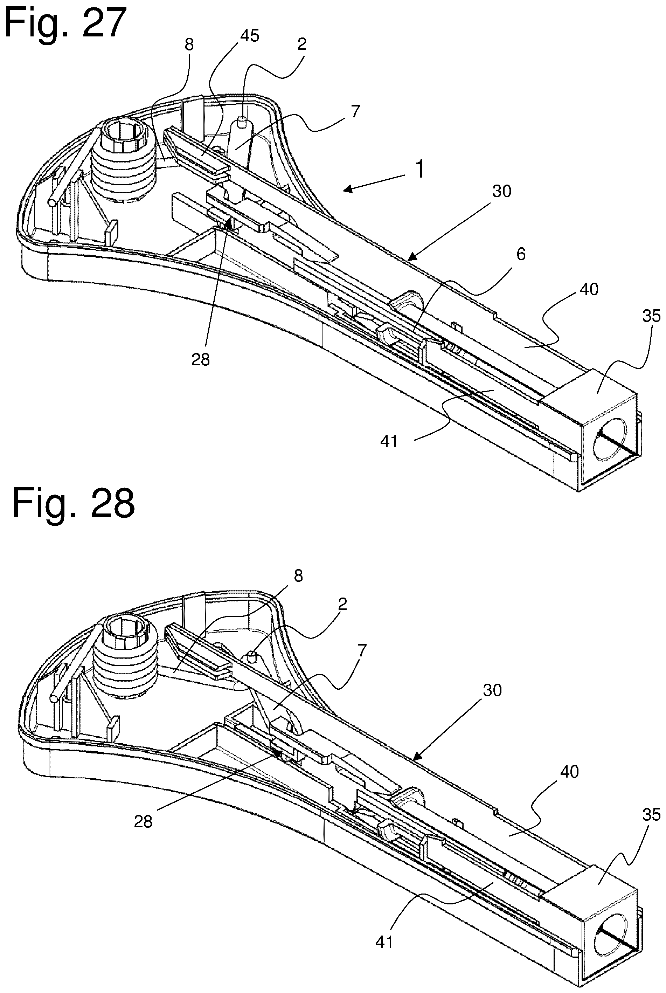

In **Figur 27** ist zunächst die Anfangsphase unmittelbar nach der Auslösung des beweglichen Federschenkels 8 nach Ausweichen der Klinke 33 gezeigt. Er hat bereits einige Winkelgrade im Uhrzeigersinn geschwenkt und nahm entsprechend den angelenkten Schwenkhebel 7 mit, der aber dadurch vom Betrachter aus gesehen im Gegenuhrzeigersinn um den mobilen Gelenkpunkt 28 schwenkt. Die **Figur 28** zeigt die Situation wenn der Federschenkel 8 etwa 30° Schwenkweg zurückgelegt hat.

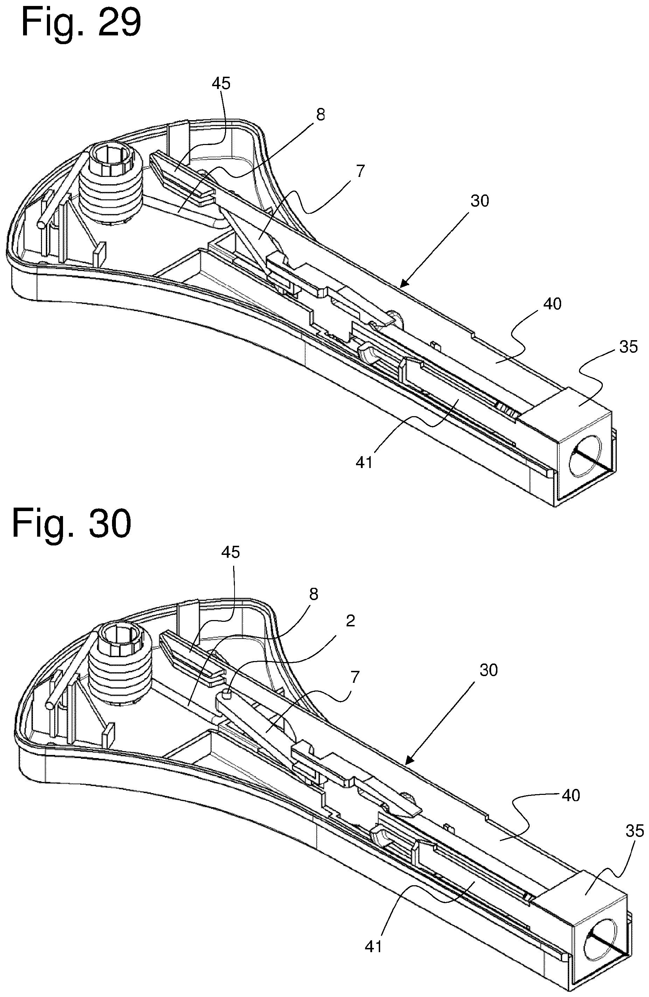

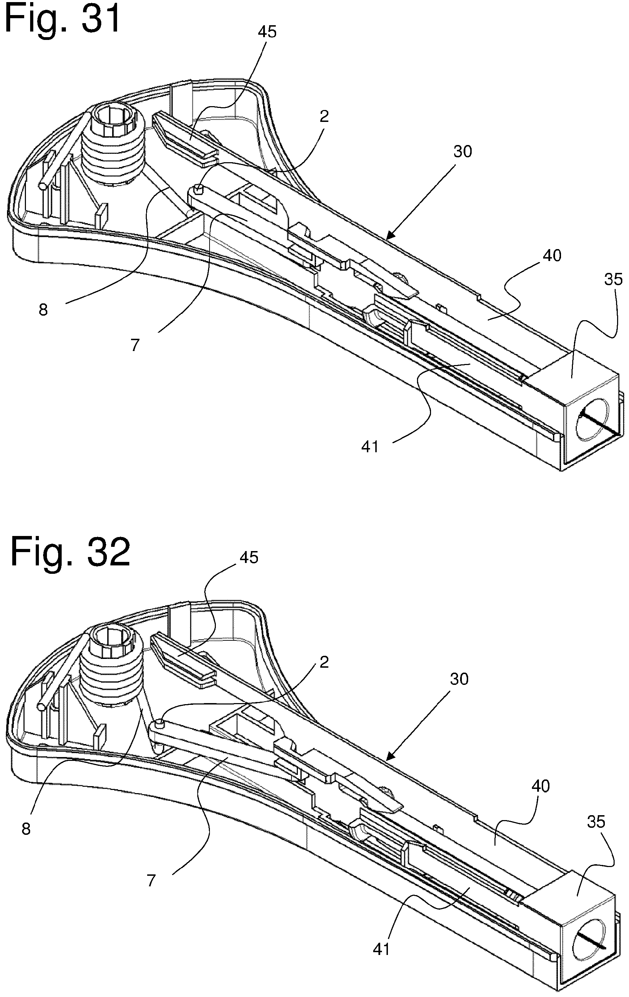

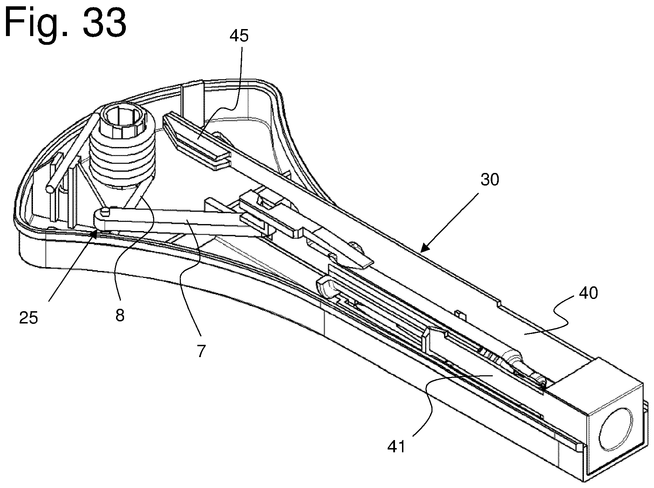

Die **Figur 29** zeigt den Selbstinjektor nach der Zurücklegung des annähernd halben Federschenkel-Weges des Federschenkels 8. In **Figur 30** ist der halbe Schwenkweg des Federschenkels 8 erreicht. Der Schwenkhebel 7 erstreckt sich jetzt fast in gleicher Richtung wie die Kolbenbewegung. In **Figur 31** hat der Federschenkel 8 ca. 2/3 des Federschenkel-Weges zurückgelegt und der Schwenkhebel 7 liegt annähernd in der Bewegungsrichtung des Kolbens. Die **Figur 32** zeigt den Zustand, wenn der Federschenkel 8 etwa 3/4 des Federschenkel-Weges zurückgelegt hat. Und schliesslich ist in **Figur 33** die Situation gezeigt, wenn der mobile Federschenkel 8 seinen Endzustand am Anschlag 25 erreichte, und entsprechend auch der an ihm angelenkte Schwenkhebel 7.

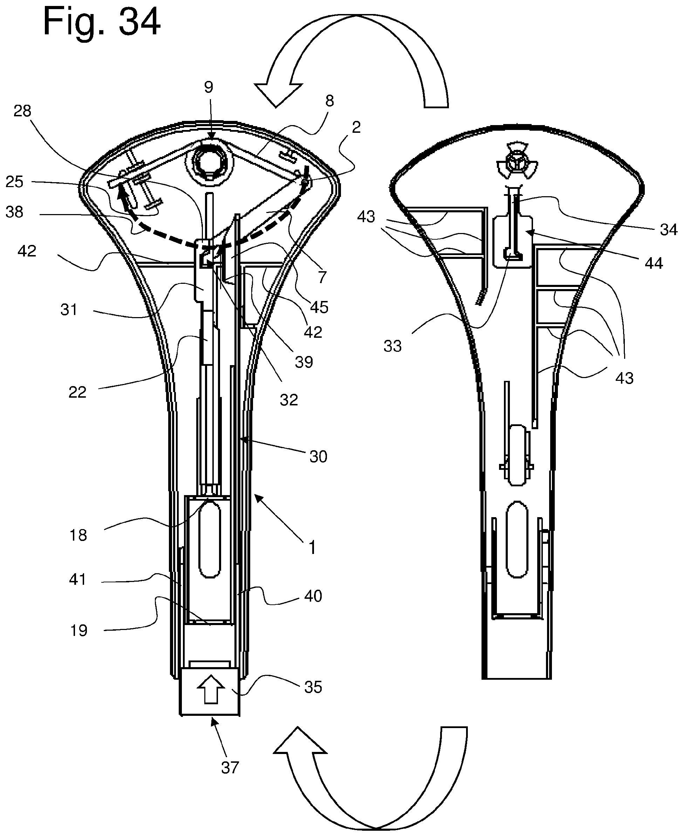

Die **Figur 34** zeigt schliesslich noch die beiden aufeinander zu klickenden Gehäuse-Teile des Selbstinjektors zur Illustration seines Auslösemechanismus , den unteren links und den oberen Gehäuseteil rechts dargestellt. Im Folgenden wird anhand dieser **Figur 34** beschrieben, wie der Auslösemechanismus im Detail funktioniert. Wenn die Spritze in den Selbstinjektor eingelegt ist, das heisst auf die beiden Stege 18, 19 mit ihren halbkreisförmigen Auflagern im unteren Gehäuseteil, dann ragt die Spritzennadel 5 in die Schutzkammer 35 und ist von derselben ganz umschlossen, ragt also nicht aus derselben heraus, obwohl die Schutzkammer 35 vorne ein kreisrundes Loch 37 aufweist und dort offen ist. In dieses Loch 37 und über die Aufsteckkappe 27 wird der Abzieher 46 mit seinem Röhrchen 47 mit seinen Widerhaken 48 aufgesteckt. Durch Abziehen kann die Aufsteckkappe 27 mit den Wiederhaken 48 des Röhrchens 47 aus dem Loch 37 vorne an der Schutzkammer 35 herausgezogen und entfernt werden. Die Nadel bleibt immer noch in der Schutzkammer 35 verborgen. Das Auslösen der Injektion erfolgt hier mittels eines Auslöse-Schiebers 30, der vorne mit der Schutzkammer 35 einstückig verbunden ist und an seinem hinteren Ende in einen vorne keilförmigen Verdränger 45 ausläuft. Wenn der Selbstinjektor im Ausgangszustand auf den Körper gedrückt wird, so wird die Schutzkammer 35 und damit auch der Auslöse-Schieber 30 im Gehäuse 1 nach hinten in das Gehäuse 1 des Selbstinjektors hineingeschoben, wie mit dem Pfeil auf der Schutzkammer 35 angezeigt. Die Schutzkammer 35 fährt mit ihrem vorderen Loch 37 über die Nadelspitze 5 nach hinten und damit wird die Nadel 5 freigelegt und kann in die Haut und den Muskel eindringen. Nun muss die Spritze 3 betätigt werden, das heisst der Kolben in der Spritze 3 muss den Wirkstoff im Spritzenkörper nach vorne und durch die Nadelspitze in den Körper fördern.

Die Auslösung geschieht folgendermassen: Die hier rechts dargestellte obere Hälfte des Gehäuses, von der hier die Innenseite sichtbar ist, ist bei zusammengesetztem Selbstinjektor wie mit den beiden gekrümmten Pfeilen angedeutet um 180° geschwenkt auf das untere und hier links dargestellte untere Gehäuseteil aufgeklickt. Dieses hier rechts dargestellte obere Gehäuseteil weist im hinteren Bereich ein Fenster bzw. eine Ausnehmung 44 auf, in welcher ein gegen links und rechts elastisch schwenkbarer Arm 34 angeformt ist, der vorne eine Klinke 33 ausformt, die im zusammengesetzten Zustand in eine Ausnehmung 32 ragt am unteren, links dargestellten Gehäuseteil hineinragt, sodass sie bei zusammengesetztem Selbstinjektor von aussen, das heisst von oben auf den oberen Gehäuseteil gesehen einsehbar ist. Das ist selbstverständlich keine Bedingung für die Funktion, sondern dient lediglich zur einfachen Herstellung der beweglichen Teile ohne Schieber im Spritz-Werkzeug. Diese Klinke 33 drückt in zusammengesetztem Zustand der beiden Gehäuseteile in die Ausnehmung 32 im hinteren, verbreiterten Anlenkteil 31 am Kolben-Schieber 22 für den Schwenkhebel 7 und blockiert ihn, das heisst verhindert eine Verschiebung des Kolben-Schiebers 22. Der Schwenkhebel 7 ist mit seinem Anlenkpunkt 2 am freien Ende des beweglichen Federschenkels 8 über den gestrichelt eingezeichneten Weg schwenkbar und mit seinem anderen Ende am Anlenkpunkt 28 gelenkig mit dem Kolben-Schieber 22 verbunden. Dieser kann sich nur längs der Schiene 11 nach vorne zur Spritze 3 hin bewegen, aber erst wenn der Kolben-Schieber 22 für eine Verschiebung nach vorne zur Spritze 3 hin freigegeben ist, das heisst wenn die Klinke 33 nicht mehr in die Ausnehmung 32 im Anlenkteil 31 des Kolben-Schiebers 22 hineinragt. Wird nun die Schutzkammer 35 infolge des Aufsetzens der Spritze mit dem daran angeformten Auslöse-Schieber 30 nach hinten geschoben, so schiebt sich der keilförmige Verdränger 45 an der elastisch hin und her schwenkbaren Klinke 33 vorbei und wenn seine hintere Kante 39 diese Klinke 33 passiert hat, so schwenkt diese mit ihrer Schrägfläche längs der Schrägfläche an der Ausnehmung 32 im Gehäuseteile wie links abgebildet von links nach rechts und somit aus der Ausnehmung 32 heraus. Der Anlenkteil 31 und damit der Kolben-Schieber 22 sind ja vorgespannt durch die Torsionsfeder, die auf den Schwenkhebel 7 wirkt und diesen nach vorne zur Spritze hin drücken will. Durch das Hinausgleiten der Klinke 33 aus der Ausnehmung 32 am Anlenkteil 31 wird dieses und somit der Kolben-Schieber 22 für eine Längsverschiebung in Richtung zur Spritze 3 hin schlagartig freigegeben. In der Folge kann die Torsionsfeder 9 mit ihrem Federschenkel 8 den daran angelenkten Schwenkhebel 7 längs der gestrichelten Halbkreislinie 38 bewegen. In der ersten Hälfte dieser Bewegung wird der Kolben-Schieber 22 kräftig zur Spritze 3 hin geschoben und betätigt den Kolben 6. In der zweiten Hälfte der Bewegung wird der Kolben-Schieber 22 zurückgezogen, bis die Bewegung des Federschenkels 8 und des Schwenkhebels 7 durch einen Anschlag 25 endet. Wie schon beschrieben wird mit dieser zweiten Bewegungshälfte die Spritze 3 selbst ein stückweit zurück in das Gehäuse 1 des Selbstinjektors hineingezogen, sodass ihre Nadel 5 in seinem Gehäuse 1 verschwindet und niemanden mehr verletzten kann. Die Rippen 44, 42 in den Gehäuseteilen sind zur deren Verstärkung vorgesehen.

Schliesslich zeigt die **Figur 35** noch eine spezielle Sicherungsplatte für die Montage dieses Selbstinjektors.. Hierzu weist die Sicherungsplatte 49 einen Zentriernocken 50 auf, sowie auf der anderen Seite einen Positionierungssteg 51. Das untere Gehäusteile des Gehäuses 1 des Selbstinjektors ist mit entsprechenden Ausnehmungen ausgeführt, die passgenau über diesen Zentriernocken 50 und Positionierungssteg 51 passen. Damit ist sichergestellt, dass der Sicherungsnocken 52 an der richtigen Stelle durch das untere Gehäuseteil in das Gehäuse 1 ragt und damit den Federschenkel 8 der Torsionsfeder blockiert. Solange der Selbstinjektor auf dieser Sicherungsplatte 49 aufliegt, ist ein Auslösung sicher verunmöglicht. Erst nach Abheben des Selbstinjektors von dieser Sicherungsplatte 49 ist er sozusagen entsichert. Nach dem Wegnehmen des Abziehers ist er entsichert und bereit für die Anwendung. Durch Aufpressen der Schutzkammer 35 auf den Körper an der Stelle, wo der Wirkstoff injiziert werden soll, wird der Auslöse-Schieber 30 ins Gehäuse 1 geschoben, legt die Nadel 5 der Spritze frei und die automatische Injektion läuft wie schon ausführlich beschrieben ab, indem der Kolben 6 in der Spritze 3 kraft der Torsionsfeder 9 nach vorne geschoben wird und hernach die Spritze 3 komplett in das Innere des Gehäuses 1 gezogen wird und damit die Nadel 5 im Gehäuse 1 verschwindet. Der Selbstinjektor ist damit gebraucht, kann niemanden mehr verletzen oder infizieren, und er kann entsorgt werden.

### Ziffernverzeichnis

- 1: Gehäuse
- 2: Anlenkpunkt Federschenkel 8 zum Schwenkhebel 7 / Kniegelenk
- 3: Spritze
- 4: Zylinder der Spritze
- 5: Nadel der Spritze
- 6: Kolben
- 7: Schwenkhebel
- 8: beweglicher Schenkel der Torsionsfeder
- 9: Torsionsfeder
- 10: Widerhaken
- 11: Schiene für Kolben
- 12: Aufschieberampe unten am Widerhaken
- 13: hinteres Ende der Spritze 3
- 14: Flansch als hinteres Ende 13 des Spritzenzylinders
- 15: Wicklungsachse Torsionsfeder
- 16: Bolzen für Torsionsfeder
- 17: stationärer Federschenkel
- 18,19: oben U-förmige Stege
- 20: Anschlag für hinteres Ende der Spritze 3
- 21: schräg abfallende Rampe am Anschlag 20
- 22: Kolben-Schieber
- 23: Rampe am Kolben-Schieber 22
- 24: Rückhalter für Torsionsfeder
- 25: Begrenzungsnocken für den Schwenkbereich des beweglichen Federschenkels
- 26: Dichtzapfen vorne am Kolben 6
- 27: Aufsteckkappe für Nadel 5
- 28: Gelenkpunkt zwischen Schwenkhebel 7 und Kolben-Schieber 22
- 29: Schenkel für Auffahrrampe
- 30: Auslöse-Schieber
- 31: Anlenkteil des Kolbenschiebers 22
- 32: Ausnehmung für Klinke 33 im Kolben-Schieber 22
- 33: Auslöse-Klinke
- 34: Elastischer Arm für die Klinke 33
- 35: Schutzkammer vorne am Auslöse-Schieber30
- 36: Unterer Rand des Kolben-Schiebers 22
- 37: Loch in Schutzkammer 35
- 38: Halbkreisline der Bewegung
- 39: hintere Kante am Verdränger 45
- 40: Schieberplatte der Schutzkammer 35 für Auslöse-Schieber 30
- 41: Führungsplatte an der Schutzkammer 35 für Auslöse-Schieber 30
- 42: Querrippen an unterem Gehäuseteil
- 43: Rippen im oberen Gehäuseteil
- 44: Fenster bzw. Ausnehmung im oberen Gehäuseteil für elastischen Arm mit Klinke 33
- 45: Verdränger
- 46: kubischer Abzieher
- 47: Röhrchen mit Widerhaken
- 48: Widerhaken im Röhrchen
- 49: Sicherungsplatte
- 50: Zentriernocken an Sicherungsplatte
- 51: Positioniersteg an Sicherungsplatte
- 52: Sicherungsnocken an Sicherungsplatte

- V: Vorderer Gehäuseteil
- H: Hinterer Gehäuseteil

## Patentansprüche

1. Selbstinjektor mit gespeicherter Energie für die Applikation eines enthaltenen flüssigen Wirkstoffes mit einem Gehäuse (1), einer Halterung (18, 19) für das Festhalten einer einzulegenden Spritze (3) mit Zylinder (4), Dichtzapfen (26) und Nadel (5), zum hydraulischen Ausstossen des Wirkstoffes durch die Nadel (5), ***dadurch gekennzeichnet, dass***
der Kolben (6) von einem längs einer Schiene (11) im Gehäuse (1) geführten Kolben-Schieber (22) durch einen an demselben angelenkten Schwenkhebel (7) betätigbar ist, und das andere Ende des Schwenkhebels (7) an einem schwenkbaren Federschenkel (8) einer vorgespannten Torsionsfeder (9) mit mehreren Windungen angelenkt ist, wobei der zweite Federschenkel (17) stationär am Gehäuse (1) arretiert ist, und die Schwenkbewegung des schwenkbaren Federschenkels (8) durch Auslösung der vorgespannten Torsionsfeder (9) auslösbar ist, wobei in einer ersten Schwenkphase mit dem schwenkenden Federschenkel (8) und dem an seinem Ende angelenkten Kolben-Schieber (22) der Kolben (6) in die Spritze (3) schiebbar ist und damit ein Widerhaken (10) am Kolben-Schieber (22) an der Spritze (3) einrastbar ist, und hernach in einer anschliessenden Schwenkbewegung des schwenkbaren Federschenkels (8) mit dem Kolben-Schieber (22) die Spritze (3) im Gehäuse (1) zurückziehbar ist.

2. Selbstinjektor nach Anspruch 1,
***dadurch gekennzeichnet, dass***
der Kolben (6) durch Beaufschlagen mit seinem Kolben-Schieber (22), der auf einer nutförmigen Schiene (11) im Gehäuse (1) geführt ist, in den Zylinder (4) der Spritze (3) einschiebbar ist, wobei am Kolben-Schieber (22) ein Widerhaken (10) mit schräg zur Verschiebachse verlaufender Aufschieberampe (12) angeformt ist, und die Spritze (3) an ihrem hinteren Ende (13) einen den Spritzenzylinder (4) radial überragenden Flansch (14) aufweist, über welchen der Widerhaken (10) mit seiner Aufschieberampe (12) elastisch ausweichend schiebbar ist und am Ende der Aufschieberrampe (12) durch elastische Rückfederung einhängt, sodass die Spritze (3) in der zweiten Schwenkphase des Schwenkhebels (7) innerhalb des Gehäuses (1) zurückziehbar ist, bis ihre Nadel (5) sich ganz innerhalb des Gehäuse (1) befindet.

3. Selbstinjektor nach einem der vorangehenden Ansprüche,
***dadurch gekennzeichnet, dass***
der Kolben (6) von einem an seinem hinteren Ende angelenkten Schwenkhebel (7) betätigbar ist, indem die Torsionsfeder (9) mit ihrer Wicklungsachse (15) auf einem Bolzen (16) innerhalb des Gehäuses (1) gelagert ist und ihr stationärer Federschenkel (17) am Gehäuse (1) abgestützt ist, während der andere, bewegliche Federschenkel (8) unter Spannung der Torsionsfeder (9) gelenkig mit dem hinteren Ende des Schwenkhebels (7) verbunden ist, und das vordere Ende des Schwenkhebels (7) gelenkig mit dem hinteren Ende des Kolben-Schiebers (22) verbunden ist, sodass bei Auslösung der Torsionsfeder (9) der Schwenkhebel (7) um den Anlenkpunkt am Kolben-Schieber (22) in einer ersten Phase für das Spritzen schwenkbar ist, bis er sich auf der Verschiebeachse des Kolbens (6) befindet, und bei Weiterschwenken in einer zweiten Schwenkphase die Spritze (3) in das Gehäuse (1) zurückzieht.

4. Selbstinjektor nach einem der vorangehenden Ansprüche,
***dadurch gekennzeichnet, dass***
die Halterung für das Festhalten der einzulegende Spritze (3) mit Zylinder (4) und Nadel (5) mindestens zwei oben U-förmig ausgenommene Stege (18, 19) aufweist, in welche der Zylinder (4) der Spritze (3) formschlüssig einpasst und dabei vom Gehäuse (1) ein Anschlag (20) gebildet ist, an den die Spritze (3) mit ihrem hinteren Flansch (14) anschlägt, sodass sie beim Einstechen der Nadel ins Gewebe nicht ins Gehäuse 1 hinein verschiebbar ist.

5. Selbstinjektor nach einem der vorangehenden Ansprüche,
***dadurch gekennzeichnet, dass***
die Halterung für das Festhalten der einzulegende Spritze (3) mit Zylinder (4) und Nadel (5) mindestens zwei oben U-förmig ausgenommene Stege (18, 19) aufweist, in welche der Zylinder (4) der Spritze (3) formschlüssig einpasst und dabei vom Gehäuse (1) ein Anschlag (20) gebildet ist, an den die Spritze (3) mit ihrem hinteren Ende und Flansch (14) anschlägt, wobei der Anschlag (20) in Richtung weg von der Spritze (3) eine schräg abfallende Rampe (21) bildet, und hinter dem Kolben (6) der Kolben-Schieber (22) an seinem unteren Rand eine nach vorne ansteigende Rampe (23) bildet, mit welcher er in der Schlussphase des Vorschiebens, wenn sein Widerhaken (10) über den hinteren Flansch (14) der Spritze fährt, auf diese Rampe (21) vor dem Anschlag (20) auffährt und damit den elastischen Schenkel (29) und den daran befindlichen Anschlag (20) verdrängt, wodurch die Spritze (3) hernach hindernisfrei vom Widerhaken (10) in das Gehäuse (1) zurückziehbar ist.

6. Selbstinjektor nach einem der vorangehenden Ansprüche,
***dadurch gekennzeichnet, dass***
er mit Ausnahme der Nadel (5), der Torsionsfeder (9) und dem Abzieh-Röhrchen (47) im Abzieher (46), die aus Metall gefertigt sind, einzig aus gespritzten Kunststoffteilen besteht.

7. Selbstinjektor nach einem der vorangehenden Ansprüche,
***dadurch gekennzeichnet, dass***
das Gehäuse (1) aus einem unteren Teil, welcher alle Teile für die Halterung und die Betätigung der Spritze (3) und ihre Zurückziehung enthält, sowie den Auslöse-Schieber (30) mit Verdränger (45) zur Auslösung, und einem darauf passenden deckungsgleichen oberen Teil mit einer daran an einem elastisch schwenkbaren Arm (34) endseitig ausgeformter und somit seitlich nachgiebiger Klinke (33) besteht, der auf den unteren Teil deckungsgleich aufklickbar ist.

8. Selbstinjektor nach einem der vorangehenden Ansprüche,
***dadurch gekennzeichnet, dass***
ein Auslöse-Schieber (30) vorne eine kubische Schutzkammer (35) für die Nadel (5) bildet und hinten in einen Verdränger (45) zur seitlichen Blockieren und Vorbeifahren an einer Auslöse-Klinke (33) ausformt, und eine seitliche Schiebeplatte (40) und eine ihr gegenüberliegende seitliche Führungspatte (41) aufweist, mittels derer der Auslöse-Schieber (30) im Gehäuse (1) parallel zur Spritze (3) geführt ist.

9. Selbstinjektor nach einem der vorangehenden Ansprüche,
***dadurch gekennzeichnet, dass***
die Bewegung des Kolbens (6) im Zylinder (4) im Ausgangszustand gesichert ist, indem die Kolben-Schiebeplatte (22) an ihrem hinteren Ende eine Ausnehmung (32) bildet, in welche eine am anderen Gehäuseteil elastisch schwenkbare Auslöse-Klinke (33) hineinragt, und wobei diese Auslöse-Klinke (33) von einem Verdränger (45), der am hinteren Ende einer Schiebestange (30) ausformt ist, und die vorne eine Schutzkammer (35) ausformt, und welche Schutzkammer (35) die Nadelspitze allseitig umschliesst, bei Druck auf die Schutzkammer (35) beim Aufsetzen des Selbstinjektors an dieser Auslöse-Klinke (33) vorbeischiebbar ist, sodass nach Passieren der Auslöse-Klinke (33) mit der hinteren Kante (39) des Verdrängers (45) diese Klinke (33) aus der Ausnehmung (32) schwenkt und damit den Kolben-Schieber (22) für eine Verschiebung zur Spritze (3) hin freigibt, sodass der Schwenkhebel (7) kraft der Torsionsfeder (9) auf die andere Seite im Gehäuse (1) schwenkbar ist.

10. Selbstinjektor nach einem der vorangehenden Ansprüche,
***dadurch gekennzeichnet, dass***
ein Abzieher (46) mit metallischem Röhrchen (47) mit nach innen ins Röhrchen (47) ragenden Widerhaken (48) über die Aufsteckkappe (27) der Nadel (5) gesteckt ist, mit welchem die Aufsteckkappe (27) durch Wegnehmen des Abziehers (46) vom Gehäuse die Nadel (5) der Spritze freilegbar ist, aber immer noch in der am Gehäuse (1) zurückschiebbaren Schutzkammer (35) verbleibt.

11. Selbstinjektor nach einem der vorangehenden Ansprüche,
***dadurch gekennzeichnet, dass***
das Gehäuse (1) in seinem vorderen Teil (V), in welchen die Spritze (3) einzulegen ist, einen im Querschnitt rechteckigen Kanal formt und sein hinterer Teil (H) von diesem Kanal aus beidseits fächerartig aufweitet und auf eine Sicherungsplatte (49) auflegbar ist, sodass eine Sicherungsnocken (52) durch eine Ausnehmung im Gehäuse (1) ragt und den Federschenkel (8) der Torsionsfeder (9) blockiert.

## Claims

1. A self-injector with stored energy for the administration of a contained liquid active substance, comprising a housing (1), a holder (18, 19) for retaining a syringe (3) to be inserted, having a barrel (4), sealing plug (26) and needle (5), for hydraulically expelling the active substance through the needle (5), **characterised in that** the piston (6) is actuatable by a piston slide (22) guided along a rail (11) in the housing (1) by means of a pivot lever (7) articulated thereto, and the other end of the pivot lever (7) is articulated to a pivotable spring leg (8) of a preloaded torsion spring (9) having multiple coils, wherein the second spring leg (17) is fixedly secured to the housing (1), and the pivoting movement of the pivotable spring leg (8) is releasable by triggering the preloaded torsion spring (9), wherein, in a first pivoting phase, with the pivoting spring leg (8) and the piston slide (22) articulated at its end, the piston (6) is displaceable into the syringe (3), thereby enabling a barb (10) on the piston slide (22) to engage with the syringe (3), and thereafter, in a subsequent pivoting movement of the pivotable spring leg (8) with the piston slide (22), the syringe (3) is retractable into the housing (1).

2. A self-injector according to claim 1, **characterised in that** the piston (6) is insertable into the barrel (4) of the syringe (3) by means of the piston slide (22), which is guided in a groove-like rail (11) in the housing (1), wherein a barb (10) having a ramp (12) inclined relative to the direction of displacement is formed on the piston slide (22), and the syringe (3) has, at its rear end (13), a flange (14) projecting radially beyond the syringe barrel (4), over which the barb (10) can slide elastically by means of its ramp (12) and, at the end of the ramp (12), engages by elastic return, such that, in the second pivoting phase of the pivot lever (7), the syringe (3) is retractable within the housing (1) until its needle (5) is completely located within the housing (1).

3. A self-injector according to any of the preceding claims, **characterised in that** the piston (6) is actuatable by a pivot lever (7) articulated at its rear end, **in that** the torsion spring (9) is mounted with its winding axis (15) on a pin (16) within the housing (1), and its stationary spring leg (17) is supported on the housing (1), while the other, movable spring leg (8) is pivotably connected under tension of the torsion spring (9) to the rear end of the pivot lever (7), and the front end of the pivot lever (7) is pivotably connected to the rear end of the piston slide (22), such that, upon release of the torsion spring (9), the pivot lever (7) is pivotable about the articulation point on the piston slide (22) in a first phase for injection until it is aligned with the displacement axis of the piston (6), and upon further pivoting, in a second phase, retracts the syringe (3) into the housing (1).

4. A self-injector according to any of the preceding claims, **characterised in that** the holder for retaining the syringe (3) comprises at least two upwardly U-shaped recessed ribs (18, 19), into which the barrel (4) of the syringe (3) fits in a form-fitting manner, and wherein the housing (1) forms a stop (20) against which the syringe (3) abuts with its rear flange (14), such that the syringe (3) cannot be displaced into the housing (1) when the needle is inserted into tissue.

5. self-injector according to any of the preceding claims, **characterised in that** the holder comprises at least two upwardly U-shaped recessed ribs (18, 19), into which the barrel (4) of the syringe (3) fits in a form-fitting manner, wherein the stop (20) forms an inclined ramp (21) extending away from the syringe (3), and the piston slide (22), behind the piston (6), forms at its lower edge a forwardly rising ramp (23), with which, in the final phase of forward movement, when its barb (10) passes over the rear flange (14) of the syringe, it rides onto the ramp (21) in front of the stop (20), thereby displacing the elastic leg (29) and the stop (20) located thereon, whereby the syringe (3) can subsequently be retracted into the housing (1) without obstruction by the barb (10).

6. A self-injector according to any of the preceding claims, **characterised in that** apart from the needle (5), the torsion spring (9), and the pull-off tube (47) in the remover (46), which are made of metal, it consists solely of injection-moulded plastic parts.

7. A self-injector according to any of the preceding claims, **characterised in that** the housing (1) consists of a lower part containing all components for holding, actuating and retracting the syringe (3), as well as a trigger slide (30) with displacer (45) for actuation, and a corresponding upper part which fits thereon and comprises a latch (33) formed at the end of an elastically pivotable arm (34) and thus laterally resilient, which can be clipped onto the lower part.

8. A self-injector according to any of the preceding claims, **characterised in that** a trigger slide (30) forms at its front end a cubic protective chamber (35) for the needle (5) and at its rear end a displacer (45) for lateral blocking and passing a trigger latch (33), and comprises a lateral sliding plate (40) and an opposite lateral guide plate (41), by means of which the trigger slide (30) is guided in the housing (1) parallel to the syringe (3).

9. A self-injector according to any of the preceding claims, **characterised in that** the movement of the piston (6) in the barrel (4) is secured in the initial state, **in that** the piston slide (22) forms at its rear end a recess (32) into which a trigger latch (33), elastically pivotable on the other housing part, projects, and wherein this trigger latch (33) can be displaced by a displacer (45), formed at the rear end of a slide rod (30), which forms at its front end the protective chamber (35) surrounding the needle tip on all sides, such that, upon pressing the protective chamber (35) when placing the self-injector, the displacer (45) passes the latch (33), and after passing it, the rear edge (39) of the displacer (45) pivots the latch (33) out of the recess (32), thereby releasing the piston slide (22) for movement towards the syringe (3), so that the pivot lever (7), under the force of the torsion spring (9), can pivot to the other side within the housing (1).

10. A self-injector according to any of the preceding claims, **characterised in that** a remover (46) with a metallic tube (47) having inwardly projecting barbs (48) is fitted over the protective cap (27) of the needle (5), by means of which the protective cap (27) can be removed from the housing by withdrawing the remover (46), thereby exposing the needle (5) of the syringe, while the needle remains within the protective chamber (35), which is retractable into the housing (1).

11. A self-injector according to any of the preceding claims, **characterised in that** the housing (1), in its front part (V) into which the syringe (3) is to be inserted, forms a channel of rectangular cross-section, and its rear part (H) widens from this channel on both sides in a fan-like manner and can be placed onto a securing plate (49), such that a securing lug (52) projects through an opening in the housing (1) and blocks the spring leg (8) of the torsion spring (9).

## Revendications

1. Auto-injecteur à énergie stockée pour l'application d'un principe actif liquide contenu, comprenant un boîtier (1), un support (18, 19) pour le maintien d'une seringue (3) à insérer avec cylindre (4), bouchon d'étanchéité (26) et aiguille (5), pour l'expulsion hydraulique du principe actif à travers l'aiguille (5), **caractérisé en ce que** le piston (6) est actionnable par un coulisseau de piston (22) guidé le long d'un rail (11) dans le boîtier (1) par l'intermédiaire d'un levier pivotant (7) articulé à celui-ci, et l'autre extrémité du levier pivotant (7) est articulée à une branche de ressort pivotante (8) d'un ressort de torsion précontraint (9) comportant plusieurs spires, la seconde branche de ressort (17) étant fixée de manière stationnaire au boîtier (1), et le mouvement de pivotement de la branche de ressort pivotante (8) étant déclenchable par libération du ressort de torsion précontraint (9), dans une première phase de pivotement, avec la branche de ressort pivotante (8) et le coulisseau de piston (22) articulé à son extrémité, le piston (6) étant déplaçable dans la seringue (3), et ainsi un crochet (10) du coulisseau de piston (22) étant encliquetable dans la seringue (3), et ensuite, dans un mouvement de pivotement suivant de la branche de ressort pivotante (8) avec le coulisseau de piston (22), la seringue (3) étant rétractable dans le boîtier (1).

2. Auto-injecteur selon la revendication 1, **caractérisé en ce que** le piston (6) est insérable dans le cylindre (4) de la seringue (3) par application de son coulisseau de piston (22), qui est guidé dans un rail en forme de rainure (11) dans le boîtier (1), un crochet (10) muni d'une rampe de poussée (12) inclinée par rapport à l'axe de déplacement étant formé sur le coulisseau de piston (22), et la seringue (3) présentant à son extrémité arrière (13) une collerette (14) dépassant radialement le cylindre de la seringue (4), au-dessus de laquelle le crochet (10) peut être poussé de manière élastique via sa rampe (12) et, à l'extrémité de la rampe (12), s'encliqueter par rappel élastique, de sorte que la seringue (3) est rétractable dans le boîtier (1) dans la seconde phase de pivotement du levier pivotant (7), jusqu'à ce que son aiguille (5) se trouve entièrement à l'intérieur du boîtier (1).

3. Auto-injecteur selon l'une quelconque des revendications précédentes, **caractérisé en ce que** le piston (6) est actionnable par un levier pivotant (7) articulé à son extrémité arrière, **en ce que** le ressort de torsion (9) est monté avec son axe d'enroulement (15) sur un axe (16) dans le boîtier (1) et que sa branche de ressort fixe (17) est appuyée sur le boîtier (1), tandis que l'autre branche de ressort mobile (8) est reliée de manière articulée, sous tension du ressort de torsion (9), à l'extrémité arrière du levier pivotant (7), et que l'extrémité avant du levier pivotant (7) est reliée de manière articulée à l'extrémité arrière du coulisseau de piston (22), de sorte que, lors de la libération du ressort de torsion (9), le levier pivotant (7) est pivotable autour du point d'articulation sur le coulisseau de piston (22) dans une première phase pour l'injection jusqu'à ce qu'il se trouve sur l'axe de déplacement du piston (6), et lors d'un pivotement ultérieur, dans une seconde phase, la seringue (3) est rétractée dans le boîtier (1).

4. Auto-injecteur selon l'une quelconque des revendications précédentes, **caractérisé en ce que** le support pour le maintien de la seringue (3) à insérer avec cylindre (4) et aiguille (5) comporte au moins deux nervures (18, 19) présentant des évidements en forme de U vers le haut, dans lesquels le cylindre (4) de la seringue (3) s'insère de manière ajustée, et que le boîtier (1) forme une butée (20) contre laquelle la seringue (3) vient en appui avec sa collerette arrière (14), de sorte qu'elle ne peut pas être déplacée dans le boîtier (1) lors de l'insertion de l'aiguille dans le tissu.

5. Auto-injecteur selon l'une quelconque des revendications précédentes, **caractérisé en ce que** le support comporte au moins deux nervures (18, 19) avec des évidements en forme de U, dans lesquels le cylindre (4) de la seringue (3) s'insère de manière ajustée, et que la butée (20) forme, en direction opposée à la seringue (3), une rampe inclinée (21), et que derrière le piston (6), le coulisseau de piston (22) forme sur son bord inférieur une rampe montante vers l'avant (23), avec laquelle, dans la phase finale du mouvement vers l'avant, lorsque son crochet (10) passe sur la collerette arrière (14) de la seringue, il monte sur la rampe (21) devant la butée (20) et déplace ainsi la branche élastique (29) et la butée (20) qui y est disposée, de sorte que la seringue (3) est ensuite rétractable sans obstacle dans le boîtier (1) par le crochet (10).

6. Auto-injecteur selon l'une quelconque des revendications précédentes, **caractérisé en ce que** à l'exception de l'aiguille (5), du ressort de torsion (9) et du tube d'extraction (47) dans l'extracteur (46), qui sont réalisés en métal, il est constitué uniquement de pièces en plastique moulées par injection.

7. Auto-injecteur selon l'une quelconque des revendications précédentes, **caractérisé en ce que** le boîtier (1) est constitué d'une partie inférieure contenant tous les éléments pour le maintien, l'actionnement et la rétraction de la seringue (3), ainsi que le coulisseau de déclenchement (30) avec organe de poussée (45) pour le déclenchement, et d'une partie supérieure correspondante, comportant un cliquet (33) formé à l'extrémité d'un bras élastiquement pivotant (34) et donc latéralement flexible, pouvant être encliquetée sur la partie inférieure.

8. Auto-injecteur selon l'une quelconque des revendications précédentes, **caractérisé en ce que** un coulisseau de déclenchement (30) forme à l'avant une chambre de protection cubique (35) pour l'aiguille (5) et à l'arrière un organe de poussée (45) pour le blocage latéral et le passage devant un cliquet de déclenchement (33), et comporte une plaque de guidage latérale (40) et une plaque de guidage opposée (41), au moyen desquelles le coulisseau de déclenchement (30) est guidé dans le boîtier (1) parallèlement à la seringue (3).

9. Auto-injecteur selon l'une quelconque des revendications précédentes, **caractérisé en ce que** le mouvement du piston (6) dans le cylindre (4) est sécurisé à l'état initial, **en ce que** le coulisseau de piston (22) forme à son extrémité arrière une cavité (32), dans laquelle un cliquet de déclenchement (33) pivotant élastiquement sur l'autre partie du boîtier pénètre, et que ce cliquet de déclenchement (33) est déplaçable par un organe de poussée (45), formé à l'extrémité arrière d'une tige coulissante (30), laquelle forme à l'avant la chambre de protection (35) entourant entièrement la pointe de l'aiguille, de sorte que, lors de la pression sur la chambre de protection (35), l'organe de poussée (45) peut être déplacé au-delà du cliquet (33), et qu'après son passage, le bord arrière (39) de l'organe de poussée (45) fait pivoter ce cliquet (33) hors de la cavité (32), libérant ainsi le coulisseau de piston (22) pour un déplacement vers la seringue (3), de sorte que le levier pivotant (7) peut pivoter sous l'effet du ressort de torsion (9).

10. Auto-injecteur selon l'une quelconque des revendications précédentes, **caractérisé en ce que** un extracteur (46) avec un tube métallique (47) comportant des crochets (48) dirigés vers l'intérieur est placé sur le capuchon (27) de l'aiguille (5), au moyen duquel le capuchon (27) peut être retiré du boîtier, exposant ainsi l'aiguille (5), tout en restant dans la chambre de protection (35).

11. Auto-injecteur selon l'une quelconque des revendications précédentes, **caractérisé en ce que** le boîtier (1), dans sa partie avant (V) destinée à recevoir la seringue (3), forme un canal de section rectangulaire, et sa partie arrière (H) s'élargit de part et d'autre en forme d'éventail et peut être posée sur une plaque de sécurité (49), de sorte qu'un ergot de sécurité (52) traverse une ouverture du boîtier (1) et bloque la branche de ressort (8) du ressort de torsion (9).
